# EUROPEAN PATENT SPECIFICATION

(11) **EP 0 701 551 B1**
(45) Date of publication and mention of the grant of the patent: **16.04.1997**
(21) Application number: 94916035.2
(22) Date of filing: 02.05.1994
(51) Int. Cl.: C07D 209/42, A61K 31/40, C07F 7/10

(54) **3-(INDOL-3-YL) PROPENOIC ACID DERIVATIVES USEFUL AS NMDA ANTAGONISTS**
3-(INDOL-3-YL) PROPENSäURDERIVATE, DIE ALS NMDA- ANTAGONISTEN NÜTZLICH SIND
DERIVES D'ACIDE 3-(INDOL-3-YL) PROPENOIQUE UTILISABLES COMME ANTAGONISTES DE NMDA

(30) Priority: 27.05.1993 US 68367; 19.10.1993 US 139323; 02.02.1994 US 190814
(43) Date of publication of application: 20.03.1996
(73) Proprietor: MERRELL PHARMACEUTICALS INC., Cincinnati, Ohio 45215-6300 (US)
(72) Inventor: SALITURO, Francesco, G., Marlborough, MA 01452 (US); BARON, Bruce, M., Cincinnati, OH 45215 (US); HARRISON, Boyd, L., Cincinnati, OH 45242 (US)
(74) Representative: VOSSIUS & PARTNER
(86) International application number: US9405023
(87) International publication number: WO9427964

(56) References cited:
- EP-A- 0 568 136
- WO-A-92/16205

## Description

The present invention is directed to a new class of excitatory amino acid antagonists. These new antagonists, 3-(indol-3-yl)-propenoic acid derivatives, are useful as NMDA (N-methyl-D-aspartate) antagonists. They preferentially bind to the strychnine-insensitive glycine binding site on the NMDA receptor complex associated with the treatment of a number of disease states. Another aspect of the invention is directed to their use in treatment of a number of diseases as well as to pharmaceutical compositions containing these excitatory amino acid antagonists.

In accordance with the present invention, a new class of NMDA antagonists have been discovered which can be described by the formula: 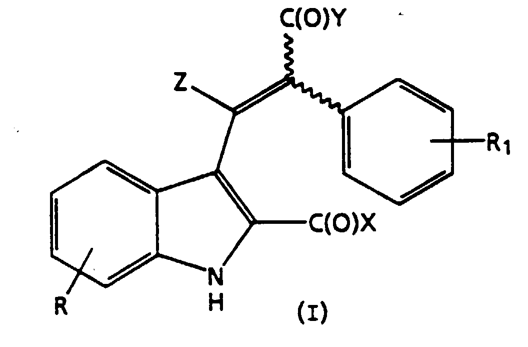 in which Z is represented by: hydrogen, -CH₃, or -C₂H₅; R is represented by from 1 to 3 substituents independently chosen from the group: hydrogen, C₁₋₄ alkyl, C₁₋₄ alkoxy, halogen, -CF₃, or -OCF₃; R₁ is represented by from 1 to 3 substituents independently chosen from the group: hydrogen, amino, C₁₋₄ alkyl, C₁₋₄ alkoxy, halogen, -CF₃, or -OCF₃; X and Y are represented by -OH, a physiologically acceptable ester, or a physiologically acceptable amide, and pharmaceutically acceptable addition salts thereof.

As used in this application:
a) the term "C₁₋₄ alkyl" refers to a branched or straight chained alkyl radical containing from 1-4 carbon atoms, such as methyl, ethyl, n-propyl, isopropyl, n-butyl, isobutyl, and the like;
b) the term "C₁₋₄ alkoxy" refers to a branched or straight chained alkoxy radical containing from 1-4 carbon atoms, such as methoxy, ethoxy, n-propoxy, isopropoxy, n-butoxy, isobutoxy, and the like;
c) the term "halogen" refers to a fluorine atom, a chlorine atom, a bromine atom, or an iodine atom;
d) the term "physiologically acceptable ester" refers to any non-toxic ester or any prodrug that allows the compounds of this application to function as NMDA antagonist: these physiologically acceptable esters may be chosen from but are not limited to compounds wherein X and Y may each independently be represented by -OR_{2,} -OCH₂OR₂ or -O-(CH₂)ₚ-NR₅R₆; R₂ is represented by C₁₋₄ alkyl, phenyl, substituted phenyl, or a phenylalkyl substituent in which the phenyl ring may be optionally substituted; R₅ and R₆ are each independently represented by a C₁₋₄ alkyl or together with the adjacent nitrogen atom form a piperidino, morpholino, or pyrrolidino group; and the pharmaceutically acceptable addition salts thereof;
e) the term "physiologically acceptable amide" refers to any non-toxic amide or any prodrug that allows the compounds of this application to function as NMDA antagonists: these physiologically acceptable amides may be chosen from, but are not limited to, compounds wherein X and Y may each independently be represented by -NR₃R₄; R₃ and R₄ are each independently represented by hydrogen, phenyl, substituted phenyl, phenylalkyl, or a C₁₋₄ alkyl; or R₃ and R₄ are taken together with the adjacent nitrogen to form a ring -CH₂-CH₂-Z-CH₂-CH₂- wherein Z is a bond, O, S, or -NR₇ in which R₇ is hydrogen or C₁₋₄ alkyl; such rings include but are not limited to piperidino, morpholino, thiomorpholino, piperazino, N-methylpiperazino, or pyrrolidino; and the pharmaceutically acceptable addition salts thereof;
f) the term "phenyl" or "Ph" refers to a phenyl moiety (C₆H₅) of the formula; 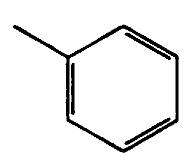
g) the term "substituted phenyl" refers to a phenyl moiety of the formula 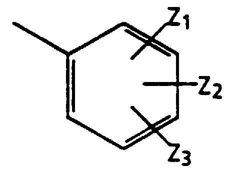 which may have from 1 to 3 substituents independently chosen from the group: hydrogen, halogens, C₁₋₄ alkyl, C₁₋₄ alkoxy, -CF₃, -OCF₃, -OH, -CN, and -NO₂. These substituents may be the same or different and may be located at any of the ortho, meta, or para positions;
h) the term "phenylalkyl substituent" or "phenylalkyl" refers to the following structure, -(CH₂)ₘ-C₆HₓZ_{y}, in which m is an integer from 1-3. This phenyl ring may be substituted in the manner described in (g);
i) the designation " 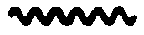 " refers to a bond for which the stereochemistry is not designated.
j) the term "pharmaceutically acceptable addition salts" refers to either an acid addition salt or a basic addition salt;

The expression "pharmaceutically acceptable acid addition salts" is intended to apply to any non-toxic organic or inorganic acid addition salt of the base compounds represented by Formula (I) or any of its intermediates. Illustrative inorganic acids which form suitable salts include hydrochloric, hydrobromic, sulfuric, and phosphoric acid and acid metal salts such as sodium monohydrogen orthophosphate, and potassium hydrogen sulfate. Illustrative organic acids which form suitable salts include the mono-, di-, and tricarboxylic acids. Illustrative of such acids are for example, acetic, glycolic, lactic, pyruvic, malonic, succinic, glutaric, fumaric, malic, tartaric, citric, ascorbic, maleic, hydroxymaleic, benzoic, hydroxybenzoic, phenylacetic, cinnamic, salicylic, 2-phenoxybenzoic, and sulfonic acids such as p-toluenesulfonic acid, methane sulfonic acid and 2-hydroxyethane sulfonic acid. Such salts can exist in either a hydrated or substantially anhydrous form. In general, the acid addition salts of these compounds are soluble in water and various hydrophilic organic solvents, and in comparison to their free base forms, generally demonstrate higher melting points.

The expression "pharmaceutically acceptable basic addition salts" is intended to apply to any non-toxic organic or inorganic basic addition salts of the compounds represented by the Formula (I) or any of its intermediates. Illustrative bases which form suitable salts include alkali metals or alkaline-earth metals hydroxides such as, sodium, potassium, calcium, magnesium, or barium hydroxides; ammonia and aliphatic, cyclic, or aromatic organic amines such as methylamine, dimethylamine, trimethylamine,and picoline.

The compounds of Formula (I) exist as geometric isomers. Any reference in this application to one of the compounds of Formula (I) is meant to encompass either a specific geometrical isomer or a mixture of isomers. The specific isomers can be separated and recovered by techniques known in the art such as chromatography, and selective crystallization.

Illustrative examples of compounds encompassed by Formula (I) include:
a) (E)-2-Phenyl-3-(2-carboethoxy-4,6-dichloroindol-3-yl)propenoic acid, ethyl ester,
b) (Z)-2-Phenyl-3-(2-carboethoxy-4,6-dichloroindol-3-yl)propenoic acid, ethyl ester,
c) (E)-2-Phenyl-3-(4,6-dichloroindol-3-yl-2-carboxylic acid)propenoic acid,
d) (Z)-2-Phenyl-3-(4,6-dichloroindol-3-yl-2-carboxylic acid)propenoic acid,
e) (E)-2-Phenyl-3-(2-carboethoxy-5,6-dichloroindol-3-yl)propenoic acid, ethyl ester,
f) (Z)-2-Phenyl-3-(2-carboethoxy-5,6-dichloroindol-3-yl)propenoic acid, ethyl ester,
g) (E)-2-Phenyl-3-(5,6-dichloroindol-3-yl-2-carboxylic acid)propenoic acid,
h) (Z)-2-Phenyl-3-(5,6-dichloroindol-3-yl-2-carboxylic acid)propenoic acid,
i) (E)-N-Methyl-2-phenyl-3-(2-carbomethylamino-4,6-dichloroindol-3-yl)propenoic amide,
j) (Z)-N-Methyl-2-phenyl-3-(2-carbomethylamino-4,6-dichloroindol-3-yl)propenoic amide,
k) (E)-2-Phenyl-3-(2-carboethoxy-6-chloroindol-3-yl)propenoic acid, ethyl ester,
l) (Z)-2-Phenyl-3-(2-carboethoxy-6-chloroindol-3-yl)propenoic acid, ethyl ester,
m) (E)-2-Phenyl-3-(6-chloroindol-3-yl-2-carboxylic acid)propenoic acid,
n) (Z)-2-Phenyl-3-(6-chloroindol-3-yl-2-carboxylic acid)propenoic acid,
o) (E)-2-(4-Methoxyphenyl)-3-(2-carboethoxy-4,6-dichloroindol-3-yl)propenoic acid, ethyl ester,
p) (Z)-2-(4-Methoxyphenyl)-3-(2-carboethoxy-4,6-dichloroindol-3-yl)propenoic acid, ethyl ester,
q) (E)-2-(4-Methoxyphenyl)-3-(4,6-dichloroindol-3-yl-2-carboxylic acid)propenoic acid,
r) (Z)-2-(4-Methoxyphenyl)-3-(4,6-dichloroindol-3-yl-2-carboxylic acid)propenoic acid,
s) (E)-2-(4-Aminophenyl)-3-(2-carboethoxy-4,6-dichloroindol-3-yl)propenoic acid, ethyl ester,
t) (Z)-2-(4-Aminophenyl)-3-(2-carboethoxy-4,6-dichloroindol-3-yl)propenoic acid, ethyl ester,
u) (E)-2-(4-Aminophenyl)-3-(4,6-dichloroindol-3-yl-2-carboxylic acid)propenoic acid,
v) (Z)-2-(4-Aminophenyl)-3-(4,6-dichloroindol-3-yl-2-carboxylic acid)propenoic acid,
w) (E)-2-(4-Chlorophenyl)-3-(2-carboethoxy-4,6-dichloroindol-3-yl)propenoic acid, ethyl ester,
x) (Z)-2-(4-Chlorophenyl)-3-(2-carboethoxy-4,6-dichloroindol-3-yl)propenoic acid, ethyl ester,
y) (E)-2-(4-Chlorophenyl)-3-(4,6-dichloroindol-3-yl-2-carboxylic acid)propenoic acid,
z) (Z)-2-(4-Chlorophenyl)-3-(4,6-dichloroindol-3-yl-2-carboxylic acid)propenoic acid,
aa) (E)-2-(4-Methylphenyl)-3-(2-carboethoxy-4,6-dichloroindol-3-yl)propenoic acid, ethyl ester,
bb) (Z)-2-(4-Methylphenyl)-3-(2-carboethoxy-4,6-dichloroindol-3-yl)propenoic acid, ethyl ester,
cc) (E)-2-(4-Methylphenyl)-3-(4,6-dichloroindol-3-yl-2-carboxylic acid)propenoic acid,
dd) (Z)-2-(4-Methylphenyl)-3-(4,6-dichloroindol-3-yl-2-carboxylic acid)propenoic acid,
ee) (E)-N-Methyl-2-phenyl-3-(4,6-dichloroindol-3-yl-2-carboxylic acid)propenoic amide,
ff) (Z)-N-Methyl-2-phenyl-3-(4,6-dichloroindol-3-yl-2-carboxylic acid)propenoic amide,
gg) (E)-N,N-Dimethyl-2-phenyl-3-(4,6-dichloroindol-3-yl-2-carboxylic acid)propenoic amide,
hh) (Z)-N,N-Dimethyl-2-phenyl-3-(4,6-dichloroindol-3-yl-2-carboxylic acid)propenoic amide,
ii) (E)-N-Phenyl-2-phenyl-3-(4,6-dichloroindol-3-yl-2-carboxylic acid)propenoic amide,
jj) (Z)-N-Phenyl-2-phenyl-3-(4,6-dichloroindol-3-yl-2-carboxylic acid)propenoic amide,
kk) (E)-N-Methyl-N-phenyl-2-phenyl-3-(4,6-dichloroindol-3-yl-2-carboxylic acid)propenoic amide,
ll) (Z)-N-Methyl-N-phenyl-2-phenyl-3-(4,6-dichloroindol-3-yl-2-carboxylic acid)propenoic amide,
mm) (E)-N-Benzyl-2-phenyl-3-(4,6-dichloroindol-3-yl-2-carboxylic acid)propenoic amide,
nn) (Z)-N-Benzyl-2-phenyl-3-(4,6-dichloroindol-3-yl-2-carboxylic acid)propenoic amide,
oo) (E)-N-Morpholino-2-phenyl-3-(4,6-dichloroindol-3-yl-2-carboxylic acid)propenoic amide,
pp) (Z)-N-Morpholino-2-phenyl-3-(4,6-dichloroindol-3-yl-2-carboxylic acid)propenoic amide,
qq) (E)-N-4-Methylpiperazino-2-phenyl-3-(4,6-dichloroindol-3-yl-2-carboxylic acid)propenoic amide,
rr) (Z)-N-4-Methylpiperazino-2-phenyl-3-(4,6-dichloroindol-3-yl-2-carboxylic acid)propenoic amide,
ss) (E)-2-Phenyl-3-(2-carboethoxy-4,6-dichloroindol-3-yl)propenoic acid,
tt) (Z)-2-Phenyl-3-(2-carboethoxy-4,6-dichloroindol-3-yl)propenoic acid,
uu) (E)-2-Phenyl-3-(2-carboethoxy-4,6-dichloroindol-3-yl)propenoic acid, t-butyl ester,
vv) (Z)-2-Phenyl-3-(2-carboethoxy-4,6-dichloroindol-3-yl)propenoic acid, t-butyl ester.

The compounds of Formula (I) can be prepared utilizing techniques that are analogously known in the art. One method of preparing these compounds is disclosed below in Reaction Scheme 1.

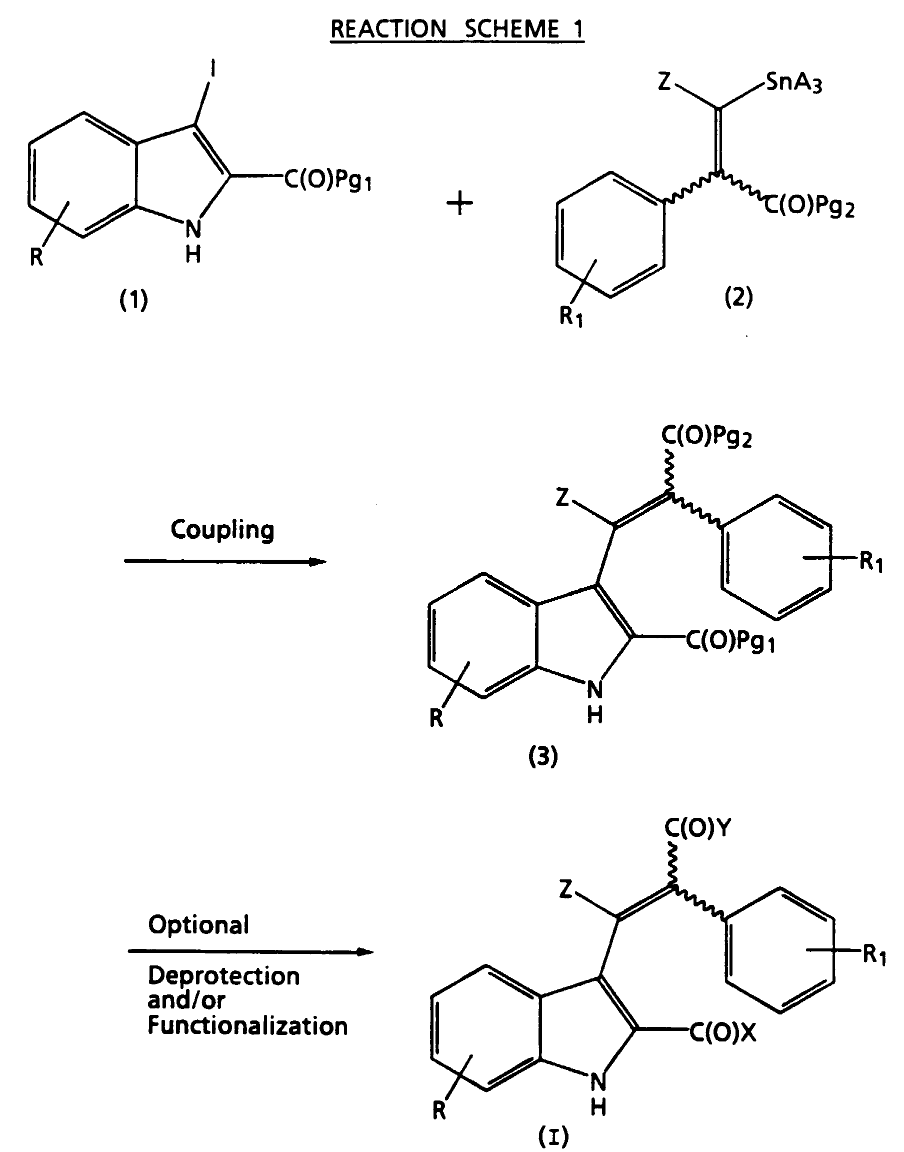

As disclosed in Reaction Scheme 1, the compounds of Formula (I) can be prepared by submitting an appropriately substituted 3-iodoindole (1) to a coupling reaction with an appropriately substituted 3-stannylpropenoic acid (2) to give compound (3). The stannyl group, SnA₃, of compound (2) will have three substituents, A, that may be either alkyl or aryl, such as phenyl, methyl, ethyl, n-butyl, etc., with methyl and n-butyl being preferred and n-butyl being most preferred. In structure (1), R is as defined for compounds of Formula (I). In structure (2) R₁ and Z are as defined for compounds of Formula (I) or give rise after deprotection to R₁ as defined for compounds of Formula (I). Pg₁ and Pg₂ are each independently represented by groups such as physiologically acceptable esters, physiologically acceptable amides, hydrolyzable esters, or active ester leaving groups known in the art.

The formation and use of active ester leaving groups is well known and appreciated in the art. Active ester leaving groups include but are not limited to anhydrides, mixed anhydrides, acid chlorides, acid bromides, 1-hydroxybenzotriazole esters, 1-hydroxysuccinimide esters, or the activated intermediates formed in the presence of coupling reagents, such as dicyclohexylcarbodiimide, 1-(3-dimethylaminopropyl)-3-ethylcarbodiimide, and 2-ethoxy-1-ethoxycarbonyl-1,2-dihydroquinoline. Active ester leaving groups may be prepared and isolated before their use or may be prepared and used without isolation to form physiologically acceptable esters or physiologically acceptable amides.

The starting materials (1) and (2) are readily available to one of ordinary skill in the art; M.R. Brennan et al., Heterocycles, 24, 2879-2884, (1986); R.D. Arnold et al., J. Org. Chem., 24, 117-118, (1959); [J.C. Cochran et al., Organometallics, 8, 804-812, (1989)] J. S. Nimitz and H. S. Mosher, Joc 46, 211-213, (1981); T. A. Blumenkopf, Synth. Commun. 16, 139-147, (1986); D. Habäch and K. Metzger, Heterocycles 24, 289-296, (1986); Y. Watanabe et al., Tet. Let. 27, 215-218 (1986); J. R. McCarthy et al., JACS 113, 7439-7440, (1991).

Typically, the coupling reaction depicted in Reaction Scheme 1 is performed in a suitable solvent, such as 1-methyl-2-pyrrolidinone. The reaction is performed using a molar excess of 3-iodoindole (1), the amount of (1) used ranges from 1.1 to 10 equivalents with 1.5 equivalents being preferred. The coupling is performed using a suitable palladium catalyst, such as tetrakis(triphenylphosphine)palladium (0), bis(acetonitrile)palladium (II) chloride, palladium (II) chloride, palladium (II) acetate, palladium (II) bromide, bis(benzonitrile)palladium (II) chloride, palladium (II) acetoacetate with tetrakis-(triphenylphosphine)palladium (0), bis(acetonitrile)palladium (II) chloride, palladium (II) chloride, palladium (II) acetate, being preferred and bis(acetonitrile)palladium (II) chloride being most preferred. The coupling is performed at a temperature ranging from 0°C to the refluxing temperature of the solvent. For couplings performed in 1-methyl-2-pyrrolidinone the preferred temperature is 60°C. The coupling reactions depicted in Reaction Scheme 1 require from 1 to 72 hours and should be stopped at a time that maximizes the desired product (3) and minimizes undesired products. The product (3) of the coupling reaction can be isolated and purified using techniques well known in the art. These techniques include aqueous extraction using suitable organic solvents, such as ethyl acetate, diethyl ether, dichloromethane, etc., evaporation, chromatography using suitable eluents, such as mixtures of ethyl acetate and hexane, dichloromethane, etc., and recrystallization.

The product (3) obtained from the coupling reaction may be optionally deprotected and/or functionalized using techniques well known in the art to give compounds of Formula (I). These techniques include hydrolysis of esters, selective hydrolysis of esters, transesterification, amidation of activated ester leaving groups, and esterification of activated ester leaving groups.

As is disclosed in Reaction Scheme 1, the compounds of Formula (I) can be prepared by submitting a compound (3) to an appropriate functionalization reaction which introduces the appropriate functionality at the 2-position of the indole nucleus and/or at the 1-position of the propenoic acid thereby producing one of the desired compounds of Formula (I). In structure (3), Z, R, and R₁ are as defined in Formula (I) or give rise after deprotection to R₁ as defined in Formula (I) and Pg₁ and Pg₂ are each independently represented by groups such as C₁₋₄ alkyl, or other active ester leaving groups known in the art, physiologically acceptable ester, or physiologically acceptable amide.

The functionalization reactions can be carried out using techniques well known in the art. For example, ester functionalities can be added to the 2-position of the indole nucleus and/or at the 1-position of the propenoic acid utilizing a variety of esterification techniques. One suitable esterification technique comprises contacting the appropriate compound of structure (3) in which Pg₁ and Pg₂ are C₁₋₄ alkyl functions with an excess of an alcohol of the formula XOH or YOH in which X and Y are the same as defined for Formula (I). The reaction is typically carried out in the presence of an excess of a base such as potassium carbonate. The reaction is typically carried out at a temperature ranging from room temperature to reflux for a period of time ranging from 1 hour to 24 hours. After the reaction is completed, the desired product of Formula (I) can be recovered by organic extraction. It may then be purified by flash chromatography and/or recrystallization as is known in the art. Suitable chromatographic solvents include mixtures of ethyl acetate and hexane, dichloromethane, and mixtures of dichloromethane and methanol. Suitable recrystallization solvents include ethyl acetate/hexane.

Amides can also be easily prepared by contacting a compound of structure (3) in which Pg₁ and Pg₂ are C₁₋₄ alkyls with an excess of ammonia or a mono- or dialkylamine corresponding to the desired X or Y substituent at a temperature of from 0-100°C for a period of time ranging from 1-48 hours using the amine as solvent or in an inert solvent such as tetrahydrofuran. The resulting amide derivatives of Formula I can then be isolated and purified by techniques known in the art.

As is readily apparent to those skilled in the art, if X and Y are not both represented by the same function in the final product, then it will be necessary to carry out the deprotection and the functionalization reactions in a sequential manner utilizing suitable protecting groups such as those described in Protecting Groups in Organic Synthesis, T. Greene. This can be done utilizing techniques known to those skilled in the art; D. B. Bryan et al., JACS, 99, 2353 (1977); E. Wuensch, Methoden der Organischen Chemie (Houben-Weyl), E. Mueller, Ed., Georg Thieme Verlag, Stuttgart, 1974, Vol. 15; M. G. Saulnierand and G. W. Gribble, JOC, 47, 2810 (1982); Y. Egawa et al., Chem. Pharm. Bull. 7, 896 (1963); R. Adams and L. H. Ulich, JACS, 42, 599 (1920); and J Szmuszkoviocz, JOC, 29, 834 (1964).

For example, a compound of Formula (I) in which Y is a physiologically acceptable amide and X is a physiologically acceptable ester or -OH can be prepared from a compound of structure (3) in which Pg₂ is t-butyl-O- and Pg₁ is a physiologically acceptable ester other than t-butyl or a hydrolyzable ester. Selective removal of the t-butyl group gives a compound of structure (3) in which Pg₂ is -OH and Pg₁ is a pysiologically acceptable ester other than t-butyl or a hydrolyzable ester which can be amidated through the formation of an activated ester leaving group followed by the addition of a suitable amine as is well known in the art. A suitable amine is one which gives a physiologically acceptable amide, Y, as is desired in the final product of Formula (I). Suitable amines include, but are not limited to, methylamine, dimethylamine, ethylamine, diethylamine, propylamine, butylamine, aniline, 4-chloroaniline, N-methylaniline, benzylamine, phenethylamine, morpholine, piperazine, piperidine, N-methylpiperazine, thiomorpholine, pyrrolidine, and N-methylbenzylamine. Formation of an active ester leaving group may require protection of the indole NH using a suitable protecting group, such as benzenesulfonyl, p-toluenesulfonyl, trimethylsilyl, trimethylsilylethoxymethyl, and the like. In cases in which the indole NH requires protection this is best done before the removal of t-butyl from Pg2. Further functionalization or hydrolysis gives a compound of Formula (I) in which Y is a physiologically acceptable amide and X is a physiologically acceptable ester or -OH. After the functionalization removal of the indole NH protecting group gives a compound of Formula (I).

Similarly, a compound of Formula (I) in which X is a physiologically acceptable amide and Y is a physiologically acceptable ester or -OH can be prepared from a compound of structure (3) in which Pg₁ is t-butyl-O- and Pg₂ is a physiologically acceptable ester other than t-butyl or a hydrolyzable ester.

The compounds of Formula (I) in which X and Y are -OH can be prepared from a compound of structure (3) in which Pg₁ and Pg₂ are C₁₋₄ alkoxy, or an activated ester leaving group by deprotection using a molar excess of a suitable reagent, such as lithium hydroxide, sodium hydroxide, potassium hydroxide, sodium bicarbonate, sodium carbonate, or potassium carbonate with lithium hydroxide, sodium hydroxide, potassium hydroxide being preferred and lithium hydroxide being most preferred. These deprotections are carried out in a suitable solvent, such as methanol, ethanol, mixtures of methanol or ethanol and water, mixtures of tetrahydrofuran and water, or water. The reaction is typically carried out at a temperature ranging from room temperature to reflux for a period of time ranging from 1 hour to 24 hours. After the reaction is completed, the desired product of Formula (I) can be recovered by techniques well known in the art, such as evaporation, precipitation by adjustment of the pH of the solution with a suitable acid such as hydrochloric acid, acetic acid, etc., extraction, and recrystallization.

The compounds of structure (2) can be prepared utilizing techniques that are analogously known in the art, J. S. Nimitz and H. S. Mosher, JOC 46, 211-213, (1981), T. A. Blumenkopf, Synth. Commun. 16, 139-147, (1986), D. Habäch and K. Metzger, Heterocycles 24, 289-296, (1986), Y. Watanabe et al., Tet. Let. 27, 215-218 (1986), J. R. McCarthy et al., JACS 113, 7439-7440, (1991). One method of preparing these compounds is disclosed below in Reaction Scheme 1a.

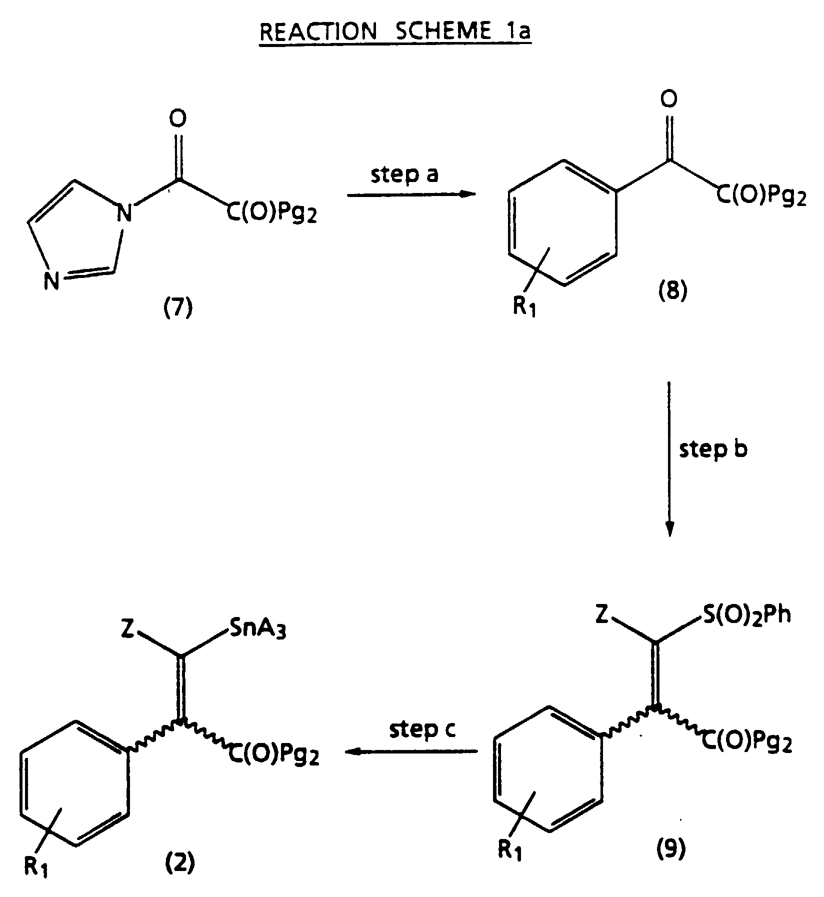

In Reaction Scheme 1a, step a, an appropriate imidazolide of structure (7) prepared by the method of J. S. Nimitz and H. S. Mosher, JOC 46, 211-213, (1981), is contacted with an appropriate organometallic reagent to give an α-keto ester of structure (8).

An appropriate imidazolide of structure (7) is one in which Pg₂ is a physiologically acceptable ester, physiologically acceptable amide, hydrolyzable ester, or active ester leaving group; or is a group which gives rise to a physiologically acceptable ester or a physiologically acceptable amide as desired in the final product of Formula (I) or gives rise to an active ester leaving group which is converted to a physiologically acceptable ester or a physiologically acceptable amide as desired in the final product of Formula (I).

An appropriate organometallic reagent is one which transfers a phenyl or substituted phenyl of the formula 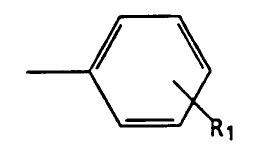 in which R₁ is as desired in the final product of the Formula (I) or gives rise upon deprotection to R₁ as desired in the final product of Formula (I).

For example, an imidazolide of structure (7) is contacted with a suitable organometallic reagent. As is appreciated by one of ordinary skill in the art a suitable organometallic reagent can be chosen from the following: organolithium reagents, organosodium reagents, organomagnesium reagents, organozinc reagents, organomanganese reagents, etc., with organolithium reagents and organomagnesium reagents being preferred and organomagnesium reagents being most preferred. The reaction is carried out in a suitable solvent, such as tetrahydrofuran or diethyl ether, at a temperature of from -78°C to the reflux temperature of the solvent. The product can be isolated by techniques well known in the art, such as extraction and evaporation in vacuo. The product can then be purified by techniques well known in the art, such as distillation, chromatography, or recrystallization.

In Reaction Scheme 1a, step b, an appropriate α-keto ester of structure (8) known in the art and known analogously in the art (J. S. Nimitz and H. S., Mosher JOC 46, 211-213, (1981)), is contacted with an appropriate organophosphorous ylide to give a vinyl sulfone of structure (9).

An appropriate α-keto ester of structure (8) is one in which Z is as desired in the final product of Formula (I); Pg₂ is a physiologically acceptable ester, physiologically acceptable amide, hydrolyzable ester, or active ester leaving group; or gives rise to an -OH, physiologically acceptable ester or a physiologically acceptable amide as desired in the final product of Formula (I); and R₁ is as desired in the final product of the Formula (I) or gives rise upon deprotection to R₁ as desired in the final product of Formula (I).

An appropriate organophosphorous ylide is one which converts an α-keto ester of structure (8) to a vinyl sulfone of structure (9). An appropriate organophosphorous ylide is formed by contacting an appropriate organophosphorous reagent, such as diethyl phenylsulphonylmethylphosphonate (T. A. Blumenkopf, Synth. Commun. 16, 139-147, (1986)), diethyl 1-(phenylsulphonyl)ethylphosphonate, or diethyl 1-(phenylsulphonyl)propylphosphonate, with a suitable base, such as lithium diisopropylamide, sodium hydride, lithium bis(trimethylsilyl)amide or potassium t-butoxide. Appropriate organophosphorous reagents and the use of appropriate organophosphorous reagents is well known and appreciated in the art (J. Boutagy and R. Thomas, Chem. Rev. 74, 8799, (1974); D. Habäch and K. Metzger, Heterocyles 24, 289-296, (1986); P. J. Kocienski and J. Tideswell, Synth. Commun. 9, 411-419, (1979)).

For example, an appropriate organophosphorous reagent is contacted with a suitable base, such as lithium diisopropylamide, sodium hydride, lithium bis(trimethylsilyl)amide or potassium t-butoxide. The ylide formation is carried out in a suitable solvent, such as tetrahydrofuran, benzene, or diethyl ether. The ylide formation is generally carried out at a temperature of from -78°C to ambient temperature. An appropriate organophosphorous ylide is contacted with an appropriate α-keto ester of structure (8). The reaction is carried out in a suitable solvent, such as tetrahydrofuran, benzene, or diethyl ether. Generally, the reaction is carried out in the same solvent used to form the appropriate organophosphorous ylide. The reaction is carried out at temperatures of from -78°C to the reflux temperature of the solvent. The reaction generally requires from 1 hour to 48 hours. The product can be isolated by techniques well known in the art, such as extraction and evaporation in vacuo. The product can then be purified by techniques well known in the art, such as distillation, chromatography, or recrystallization.

In Reaction Scheme 1a, step c, an appropriate vinyl sulfone of structure (9) is contacted with an appropriate tri-substituted tin hydride reagent to give an appropriately substituted 3-stannylpropenoic acid (2).

An appropriate vinyl sulfone of structure (9) is one in which Z, R₁, and Pg₂ are as is desired on the final product of Formula (I) or is one in which R₁ and Pg₂ give rise after deprotection and/or functionalization to R₁ and Y as are desired in the final product of Formula (I).

An appropriate tri-substituted tin hydride reagent is one which introduces the stannyl group, SnA₃. An appropriate tri-substituted tin hydride reagent has three substituents, A, which may be either alkyl or aryl, such as phenyl, methyl, ethyl, n-butyl, etc., with methyl and n-butyl being preferred and n-butyl being most preferred.

For example, an appropriate vinyl sulfone of structure (9) is contacted with from 2 to 5 molar equivalents of an appropriate tri-substituted tin hydride reagent. The reaction is carried out in a suitable solvent, such as toluene, benzene, hexane, or cyclohexane. The reaction is carried out in the presence of a suitable catalyst, such as 2,2'-azobisisobutyronitrile (AIBN), benzoyl peroxide, and the like. The reaction is carried out at a temperature from ambient temperature to the refluxing temperature of the solvent. Compounds of the structure (2) can be isolated and purified by techniques well known in the art, such as extraction, evaporation, chromatography, and recrystallization.

Another method of preparing the compounds of Formula (I) is disclosed below in Reaction Scheme 2.

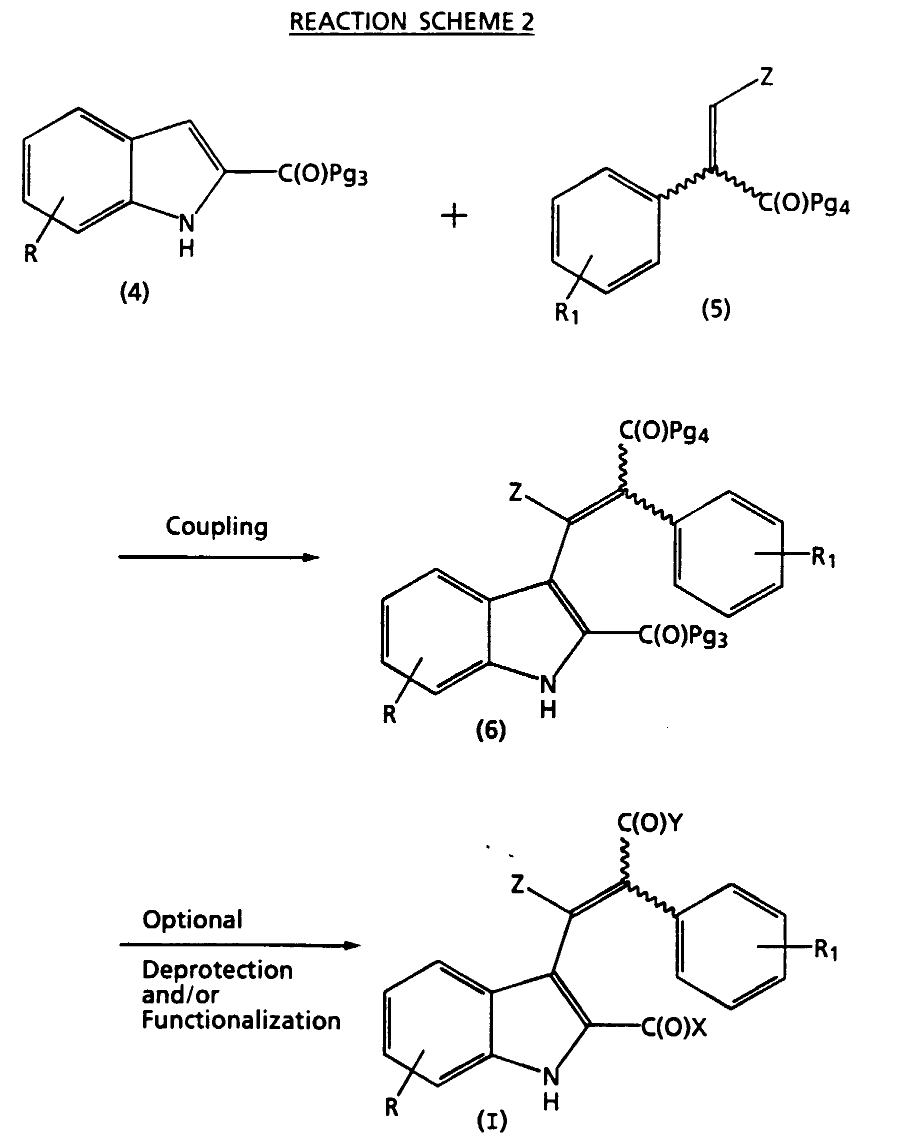

As disclosed in Reaction Scheme 2, the compounds of Formula (I) can be prepared by submitting an appropriately substituted indole (4) to a coupling reaction with an appropriately substituted propenoic acid derivative (5) to give compound (6). In structure (4), R is as defined for compounds of Formula (I). In structure (5) R₁ and Z are as defined for compounds of Formula (I) or give rise after deprotection to a group R₁ as desired in the final product of Formula(I). Pg₃ and Pg₄ are each independently represented by groups such as physiologically acceptable esters, physiologically acceptable amides, hydrolyzable esters, or active ester leaving groups known in the art. The starting materials (4) and (5) and techniques used in Reaction Scheme 2 are readily available to one of ordinary skill in the art; I. Amer and H. Alper, J. Organometallic Chem., 383, 573-77, (1990); C. Nájera et al., Tet. Let., 30, 6085-88, (1989); Y. Murakami et al., Heterocycles, 22, 1493-96, (1984); J.C. Cochran et al., Organometallics, 8, 804-812, (1989). The coupling reaction of Reaction Scheme 2 is preferred when compounds of Formula (I) in which Y is a physiologically acceptable amide are to be prepared directly in the coupling reaction. Typically, the coupling reaction depicted in Reaction Scheme 2 is performed in a suitable solvent, such as acetic acid, trifluoroacetic acid, acetonitrile, methanol, dimethylformamide with trifluoroacetic acid being preferred. The reaction is performed using an approximately one to one ratio of starting materials (4) and (5). The coupling is performed using an equimolar amount or a slight molar excess of a suitable palladium reagent, such as bis(acetonitrile)palladium (II) chloride, palladium (II) chloride, palladium (II) acetate, palladium (II) bromide, palladium (II) trifluoroacetate, bis(benzonitrile) palladium (II) chloride, palladium (II) acetoacetate with bis(acetonitrile)palladium (II) chloride, palladium (II) trifluoroacetate, palladium (II) chloride, palladium (II) acetate being preferred and with palladium (II) chloride and palladium (II) trifluoroacetate being most preferred. The coupling is performed at a temperature ranging from 0°C to the refluxing temperature of the solvent. For couplings performed in trifluoroacetic acid the preferred temperature is 50°C. The coupling reactions depicted in Reaction Scheme 2 require from 1 to 72 hours and should be stopped at a time that maximizes the desired product (6) and minimizes undesired products. The product (6) of the coupling reaction can be isolated and purified using techniques well known in the art. These techniques include: aqueous extraction using suitable organic solvents, such as ethyl acetate, diethyl ether, dichloromethane, etc., evaporation, chromatography using suitable eluents, such as mixtures of ethyl acetate and hexane, dichloromethane, etc., and recrystallization.

The compound (6) may be optionally deprotected and/or functionalized using techniques well known in the art to give compounds of Formula (I) as was taught above in Reaction Scheme 1 for compounds of structure (3).

The compounds of Formula (I) are excitatory amino acid antagonists. They antagonize the effects which excitatory amino acids have upon the NMDA receptor complex. They preferentially bind to the strychnine-insensitive glycine binding site on the NMDA receptor complex associated with the treatment of a number of disease states. See Palfreyman, M.G. and B.M. Baron, Excitatory Amino Acid Antagonists, B.S. Meldrum ed., Blackwell Scientific, 101-129 (1991); and, Kemp, J.A., and P.D. Leeson, Trends in Pharmacological Sciences, 14, 20-25 (1993).

The compounds exhibit anticonvulsant properties and are useful in the treatment of grand mal seizures, petit mal seizures, psychomotor seizures, autonomic seizures, etc. One method of demonstrating their antiepileptic properties is by their ability to inhibit the seizures that are caused by the administration of quinolinic acid. This test can be conducted in the following manner.

One group containing ten mice are administered 0.01-100 micrograms of test compound intracerebroventricularly in a volume of 5 microliters of saline. A second control group containing an equal number of mice are administered an equal volume of saline as a control. Approximately 5 minutes later, both groups are administered 7.7 micrograms of quinolinic acid intracerebroventricularly in a volume of 5 microliters of saline. The animals are observed for 15 minutes thereafter for signs of tonic seizures. The control group will have a statistically higher rate of tonic seizures than will the test group.

Another method of demonstrating the antiepileptic properties of these compounds is by their ability to inhibit audiogenic convulsions in DBA/2J mice. This test can be conducted in the following manner. Typically one group of from 6-8 male DBA/2J mice are administered from about 0.01 micrograms to about 10 micrograms of the test compound. The test compound is administered into the lateral ventricle of the brain or intraperitoneally. A second group of mice are administered an equal volume of a saline control by the same route. Five minutes later the mice are placed individually in glass jars and are exposed to a sound of 110 decibels for 30 seconds. Each mouse is observed during the sound exposure for signs of seizure activity. The control group will have a statistically higher incidence of seizures than the group which receives the test compound.

The compounds of Formula (I) are useful for preventing or minimizing the damage which nervous tissues contained within the CNS suffer upon exposure to either ischemic, traumatic, or hypoglycemic conditions including strokes or cerebrovascular accidents, cardiovascular surgery, concussions, hyperinsulinemia, cardiac arrest, drownings, suffocations, and neonatal anoxic trauma. The compounds should be administered to the patient within 24 hours of the onset of the hypoxic, ischemic, traumatic, or hypoglycemic condition in order to minimize the CNS damage which the patient will experience.

The compounds are also useful in the treatment of neurodegenerative diseases such as Huntington's disease, Alzheimer's disease, senile dementia, glutaric acidaemia type I, multi-infarct dementia, amyotrophic lateral sclerosis, and neuronal damage associated with uncontrolled seizures. The administration of these compounds to a patient experiencing such a condition will serve to either prevent the patient from experiencing further neurodegeneration or it will decrease the rate at which the neurodegeneration occurs.

As is apparent to those skilled in the art, the compounds will not correct any CNS damage that has already occurred as the result of either disease, physical injury, or a lack of oxygen or sugar. As used in this application, the term "treat" refers to the ability of the compounds to prevent further damage or delay the rate at which any further damage occurs.

The compounds exhibit an anxiolytic effect and are thus useful in the treatment of anxiety. These anxiolytic properties can be demonstrated by their ability to block distress vocalizations in rat pups. This test is based upon the phenomenon that when a rat pup is removed from its litter, it will emit an ultrasonic vocalization. It was discovered that anxiolytic agents block these vocalizations. The testing methods have been described by Gardner, C.R., *Distress Vocalization in Rat Pups: A Simple Screening Method For Anxiolytic Drugs,* J. Pharmacol. Methods, 14, 181-87 (1986) and Insel et al., *Rat Pup Isolation Calls: Possible Mediation by the Benzodiazepine Receptor Complex,* Pharmacol. Biochem. Behav., 24, 1263-67 (1986).

The compounds also exhibit an analgesic effect and are useful in controlling pain. The compounds are also effective in the treatment of migraine.

In order to exhibit these therapeutic properties, the compounds need to be administered in a quantity sufficient to inhibit the effect which the excitatory amino acids have upon the NMDA receptor complex. The dosage range at which these compounds exhibit this antagonistic effect can vary widely depending upon the particular disease being treated, the severity of the patient's disease, the patient, the particular compound being administered, the route of administration, and the presence of other underlying disease states within the patient, etc. Typically the compounds exhibit their therapeutic effect at a dosage range of from about 0.1 mg/kg/day to about 50 mg/kg/day for any of the diseases or conditions listed above. Repetitive daily administration may be desirable and will vary according to the conditions outlined above.

The compounds of the present invention may be administered by a variety of routes. They are effective if administered orally. The compounds may also be administered parenterally (i.e. subcutaneously, intravenously, intramuscularly, intraperitoneally, or intrathecally).

Pharmaceutical compositions can be manufactured utilizing techniques known in the art. Typically a therapeutic amount of the compound will be admixed with a pharmaceutically acceptable carrier.

For oral administration, the compounds can be formulated into solid or liquid preparations such as capsules, pills, tablets,- lozenges, melts, powders, suspensions, or emulsions. Solid unit dosage forms can be capsules of the ordinary gelatin type containing, for example, surfactants, lubricants and inert fillers such as lactose, sucrose, and cornstarch or they can be sustained release preparations.

In another embodiment, the compounds of Formula (I) can be tableted with conventional tablet bases such as lactose, sucrose, and cornstarch, in combination with binders, such as acacia, cornstarch, or gelatin, disintegrating agents such as potato starch or alginic acid, and a lubricant such as stearic acid or magnesium stearate. Liquid preparations are prepared by dissolving the active ingredient in an aqueous or nonaqueous pharmaceutically acceptable solvent which may also contain suspending agents, sweetening agents, flavoring agents, and preservative agents as are known in the art.

For parenteral administration the compounds may be dissolved in a physiologically acceptable pharmaceutical carrier and administered as either a solution or a suspension. Illustrative of suitable pharmaceutical carriers are water, saline, dextrose solutions, fructose solutions, ethanol, or oils of animal, vegetative, or synthetic origin. The pharmaceutical carrier may also contain preservatives, buffers, etc., as are known in the art. When the compounds are being administered intrathecally, they may also be dissolved in cerebrospinal fluid as is known in the art.

The compounds of this invention can also be administered topically. This can be accomplished by simply preparing a solution of the compound to be administered, preferably using a solvent known to promote transdermal absorption such as ethanol or dimethyl sulfoxide (DMSO) with or without other excipients. Preferably topical administration will be accomplished using a patch either of the reservoir and porous membrane type or of a solid matrix variety.

Some suitable transdermal devices are described in U.S. Pat. Nos. 3,742,951; 3,797,494; 3,996,934; and 4,031,894. These devices generally contain a backing member which defines one of its face surfaces, an active agent permeable adhesive layer defining the other face surface and at least one reservoir containing the active agent interposed between the face surfaces. Alternatively, the active agent may be contained in a plurality of microcapsules distributed throughout the permeable adhesive layer. In either case, the active agent is delivered continuously from the reservoir or microcapsules through a membrane into the active agent permeable adhesive, which is in contact with the skin or mucosa of the recipient. If the active agent is absorbed through the skin, a controlled and predetermined flow of the active agent is administered to the recipient. In the case of microcapsules, the encapsulating agent may also function as the membrane.

In another device for transdermally administering the compounds in accordance with the present invention, the pharmaceutically active compound is contained in a matrix from which it is delivered in the desired gradual, constant and controlled rate. The matrix is permeable to the release of the compound through diffusion or microporous flow. The release is rate controlling. Such a system, which requires no membrane is described in U.S. Pat. No. 3,921,636. At least two types of release are possible in these systems. Release by diffusion occurs when the matrix is nonporous. The pharmaceutically effective compound dissolves in and diffuses through the matrix itself. Release by microporous flow occurs when the pharmaceutically effective compound is transported through a liquid phase in the pores of the matrix.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art.
As used in this application:
k) the term "patient" refers to warm blooded animals such as, for example guinea pigs, mice, rats, cats, rabbits, dogs, monkeys, chimpanzees, and human;
l) the term "treat" refers to the ability of the compounds to either relieve, alleviate, or slow the progression of the patient's disease;
m) the term "neurodegeneration" refers to a progressive death and disappearance of a population of nerve cells occurring in a manner characteristic of a particular disease state and leading to brain damage.

The compounds of Formula (I) may also be admixed with any inert carrier and utilized in laboratory assays in order to determine the concentration of the compound within the serum, urine, etc., of the patient as is known in the art.

Neurodegeneration diseases are typically associated with a loss of NMDA receptors. Thus, the compounds of Formula (I) may be utilized in diagnostic procedures to aid physicians with the diagnosis of neurodegenerative diseases. The compounds may be labeled with imaging agents known in the art such as isotopic ions and administered to a patient in order to determine whether the patient is exhibiting a decreased number of NMDA receptors and the rate at which that loss is occurring.

The following preparations represent typical procedures as described in Reaction Scheme la, for preparing starting materials used in the examples. The following examples present typical syntheses as described in Reaction Scheme 1, and Reaction Scheme 2. These preparations and examples are understood to be illustrative only and are not intended to limit the scope of the invention in any way. As used in the following preparations and examples, the following terms have the meanings indicated: "g" refers to grams, "mg" refers to milligrams, "mmol" refers to millimoles, "mL" refers to milliliters, "°C" refers to degrees Celsius, "M" refers to molar, "mp" refers to melting point, "dec" refers to decomposition, "THF" refers to tetrahydrofuran, "R_{f}" refers to retention factor.

### PREPARATION 1

### 4,6-Dichloro-3-iodoindole-2-carboxylic acid, ethyl ester

Combine 4,6-dichloro-indole-2-carboxylic acid, ethyl ester (5.2 g, 20.0 mmol) and sodium hydroxide (0.80 g, 20 mmol) in ethanol (250 mL). Add iodine (5.1 g, 20.0 mmol) as a solution in ethanol (100 mL). After 1 hour, concentrate the reaction mixture in vacuo to obtain a residue. Dissolve the residue in ethyl acetate and extract with 1 M hydrochloric acid solution and then with saturated sodium chloride solution. Dry the organic layer over magnesium sulfate and evaporate in vacuo. Chromatograph on silica gel eluting with 25% ethyl acetate/hexane. Combine the product containing fractions and evaporate in vacuo. Recrystallize form ethyl acetate/hexane to give the title compound as a solid; mp: 218-220°C. Elem. Anal. calculated for C₁₁H₈Cl₂INO₂: C, 34.41; H, 2.10; N, 3.65. Found: C, 34.67; H, 2.09; N, 3.68.

### PREPARATION 2

### Ethyl 4-N,N'-(1,1,4,4-tetramethyl-1,4-disilethyleneamino)benzoylformate

Combine magnesium turnings (12 mmol) and 1,2-dibromoethane (2 mmol) in anhydrous diethyl ether (500 mL). Heat to a gentle reflux and slowly add a solution of 4-bromo-N,N'-(1,1,4,4-tetramethyl-1,4-disilethylene)aniline (T. L. Guggenheim, Tet. Let. 25, 1253-1254 (1984)) (10 mmol) in diethyl ether (100 mL). Heat until the magnesium turnings have reacted. Cool to 0°C. Add ethyl α-oxo-1H-imidazole-1-acetate (J. S. Nimitz and H. S. Mosher, JOC 46, 211-213, (1981)) (10 mmol). After the addition is complete, warm to ambient temperature. After 18 hours, pour the reaction mixture into a cold ammonium chloride solution. Extract with ethyl acetate and combine the organic layers. Dry over magnesium sulfate and evaporate in vacuo. Chromatograph on silica gel to give the title compound.

### PREPARATION 3 a) 2-Phenyl-3-(tri-n-butylstannyl)propenoic acid, methyl ester

Combine diethyl phenylsulphonylmethylphosphonate (T. A. Blumenkopf, Synth. Commun. 16, 139-147, (1986)) (117.0 g, 400 mmol) and tetrahydrofuran (500 mL). Cool in an ice-bath. Add lithium bis(trimethylsilyl)amide (480 mL, 1 M in tetrahydrofuran, 480 mmol). Stir for 30 minutes after the addition of lithium bis(trimethylsilyl)amide, then add methyl benzoylformate (72.0 g, 439 mmol). Warm to ambient temperature and stir for 2 hours. Partition between water and ethyl acetate. Extract the aqueous layer with ethyl acetate. Combine the organic layers, dry over magnesium sulfate and evaporate in vacuo to give an oil. Triturate with cyclohexane to give a solid. Chromatograph the solid on silica gel eluting sequentially with 10% ethyl acetate/hexane, 15% ethyl acetate/hexane, and 33% ethyl acetate/hexane. Evaporation of the product containing fractions gives a solid. Recrystallize from ethyl acetate/hexane to give 2-phenyl-3-sulfonylphenyl-propenoic acid, methyl ester as a solid. Elem. Anal calculated for C₁₆H₁₄O₄S: C, 63.56; H, 4.67. Found: C, 63.25; H, 4.70.

Combine 2-phenyl-3-sulfonylphenyl-propenoic acid, methyl ester (41.0 g, 136 mmol) and tri-n-butyltin hydride (79.0 g, 271 mmol) in cyclohexane (900 mL). Add 2,2'-azobisisobutyronitrile (AIBN) (0.7 g, 4.3 mmol) and heat to reflux for 3 hours. Cool to ambient temperature and evaporate in vacuo. Chromatograph on silica gel eluting with 20% ethyl acetate/hexane. Evaporate the product containing fractions and chromatograph on silica gel eluting sequentially with 5% ethyl acetate/hexane and 10% ethyl acetate/hexane to give the title compound as a clear oil.
b) 2-Phenyl-3-(tri-n-butylstannyl)propenoic acid, ethyl ester can be prepared by the method of Preparation 3 by using ethyl benzoylformate (J. S. Nimitz and H. S. Mosher, JOC 46, 211-213, (1981)).
c) 2-(4-Methylphenyl)-3-(tri-n-butylstannyl)propenoic acid, t-butyl ester can be prepared by the method of Preparation 3 by using t-butyl 4-methylbenzoylformate (J. S. Nimitz and H. S. Mosher, JOC 46, 211-213, (1981)).
d) 2-(4-Chlorophenyl)-3-(tri-n-butylstannyl)propenoic acid, t-butyl ester can be prepared by the method of Preparation 3 by using t-butyl 4-chlorobenzoylformate (J. S. Nimitz and H. S. Mosher, JOC 46, 211-213, (1981)).
e) 2-(4-Methoxyphenyl)-3-(tri-n-butylstannyl)propenoic acid, t-butyl ester can be prepared by the method of Preparation 3 by using t-butyl 4-methoxybenzoylformate (J. S. Nimitz and H. S. Mosher, JOC 46, 211-213, (1981)).
f) 2-N,N'-(1,1,4,4-Tetramethyl-1,4-disilethyleneaminophenyl)-3-(tri-n-butylstannyl)propenoic acid, t-butyl ester can be prepared by the method of Preparation 3 by using t-butyl 4-N,N'-(1,1,4,4-tetramethyl-1,4-disilethyleneamino)benzoylformate prepared as in Preparation 2 using t-butyl benzoylformate (J. S. Nimitz and H. S. Mosher, JOC 46, 211-213, (1981)).
g) 2-(4-Methylphenyl)-3-(tri-n-butylstannyl)propenoic acid, ethyl ester can be prepared by the method of Preparation 3 by using ethyl 4-methylbenzoylformate (J. S. Nimitz and H. S. Mosher, JOC 46, 211-213, (1981)).
h) 2-(4-Chlorophenyl)-3-(tri-n-butylstannyl)propenoic acid, ethyl ester can be prepared by the method of Preparation 3 by using ethyl 4-chlorobenzoylformate (J. S. Nimitz and H. S. Mosher, JOC 46, 211-213, (1981)).
i) 2-(4-Methoxyphenyl)-3-(tri-n-butylstannyl)propenoic acid, ethyl ester can be prepared by the method of Preparation 3 by using ethyl 4-methoxybenzoylformate (J. S. Nimitz and H. S. Mosher, JOC 46, 211-213, (1981)).
j) 2-4-N,N'-(1,1,4,4-Tetramethyl-1,4-disilethyleneaminophenyl)-3-(tri-n-butylstannyl)propenoic acid, ethyl ester can be prepared by the method of Preparation 3 by using ethyl 4-N,N'-(1,1,4,4-tetramethyl-1,4-disilethyleneamino)benzoylformate as prepared in Preparation 2.

### EXAMPLE 1

### (E) and (Z)-2-Phenyl-3-(2-carboethoxy-4,6-dichloroindol-3-yl)propenoic acid, ethyl ester

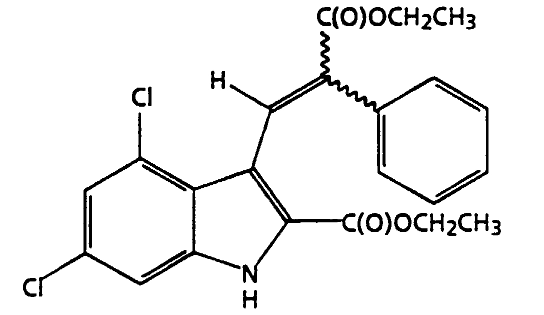

Combine 4,6-dichloro-3-iodoindole-2-carboxylic acid, ethyl ester (1.5g, 3.9 mmol) and 2-phenyl-3-(tri-n-butylstannyl)propenoic acid, ethyl ester (1.1g, 2.4 mmol) in 1-methyl-2-pyrrolidinone (5 mL) and add bis-(acetonitrile)palladium (II) dichloride (2.07 mg, 0.08 mmol). Flush the vessel with nitrogen gas, seal and heat to 60°C. After stirring for 8 hours add more bis-(acetonitrile)palladium (II) dichloride (2.07 mg, 0.08 mmol) and continue stirring for 16 hours. Pour the reaction mixture into water and extract with diethyl ether, dry over magnesium sulfate and evaporate in vacuo. Chromatograph on silica gel eluting with 15% ethyl acetate/hexane to give 0.60 g of the title compound; mp: 179-182°C. Elem. Anal. calculated for C₂₂H₁₉C₁₂NO₄: C, 61.12; H, 4.43; N, 3.24. Found: C, 60.84; H, 4.33; N, 3.17.

### EXAMPLE 2

### (E) and (Z)-2-Phenyl-3-(2-carboethoxy-4,6-dichloroindol-3-yl)propenoic acid, ethyl ester

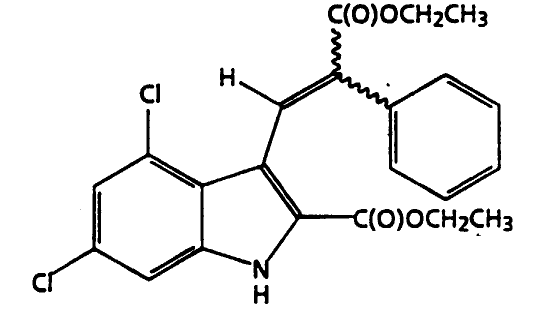

Combine 4,6-dichloroindole-2-carboxylic acid, ethyl ester (1.50 g, 4.0 mmol) and palladium (II) diacetate (0.90 g, 4.0 mmol) in trifluoroacetic acid (5 mL) and heat at 50°C for 0.5 hours to dissolve the solids. Add 2-phenylpropenoic acid, ethyl ester (1.5g, 8.5 mmol) and stir at 50°C for 16 hours. Remove the solvent by evaporation in vacuo and dilute the residue with ethyl acetate and extract with saturated sodium bicarbonate solution. Chromatograph on silica gel eluting with 15% ethyl acetate/hexane to give 0.40 g of the title compound; mp: 164-168°C. Elem. Anal. calculated for C₂₂H₁₉Cl₂NO₄: C, 61.12; H, 4.43; N, 3.24. Found: C, 60.82; H, 4.43; N, 3.16.

### EXAMPLE 3

### (E)-2-Phenyl-3-(4,6-dichloroindol-3-yl-2-carboxylic acid)-propenoic acid

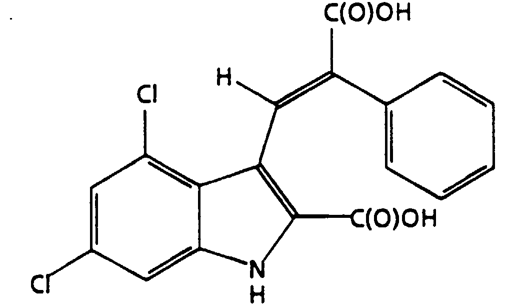

Combine (E) and (Z)-2-phenyl-3-(2-carboethoxy-4,6-dichloroindol-3-yl)propenoic acid, ethyl ester (65 mg, 0.16 mmol) and lithium hydroxide (20.2 mg, 0.48 mmol) in THF/water (3 mL, 1/1) and heat to 50°C for 24 hours. Dilute the reaction mixture with water (5 mL) and acidify with 1M hydrochloric acid. Extract with ethyl acetate and dry the organic layer with magnesium sulfate. Evaporate in vacuo and recrystallize the residue from ethyl acetate/hexane to give 57 mg of the title compound; mp: 268-270°C (dec). Elem. Anal. calculated for C₁₈H₁₁Cl₂NO₄(0.5 ethyl acetate): C, 57.16; H, 3.60; N, 3.33. Found: C, 56.95; H, 3.67; N, 3.29.

### EXAMPLE 4

### (E) and (Z)-2-Phenyl-3-(2-carboethoxy-5,6-dichloroindol-3-yl)propenoic acid, ethyl ester

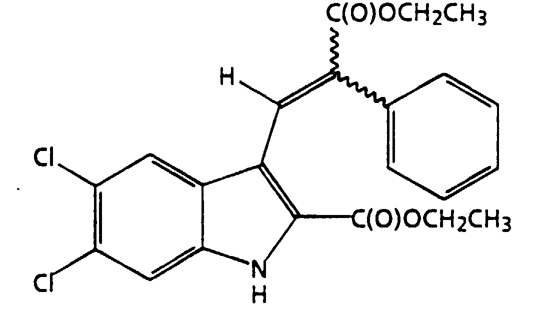

Combine 5,6-dichloro-3-iodoindole-2-carboxylic acid, ester ester (1.5g, 3.9 mmol) and 2-phenyl-3-(tri-n-butylstannyl)propenoic acid, ethyl ester (1.1 g, 2.4 mmol) in 1-methyl-2-pyrrolidinone (5 mL) and add bis-(acetonitrile)palladium (II) dichloride (2.07 mg, 0.08 mmol). Flush the vessel with nitrogen gas, seal and heat to 60°C. After stirring for 5 hours pour the reaction mixture into water and extract with diethyl ether, dry over magnesium sulfate and evaporate in vacuo. Chromatograph on silica gel eluting with 15% ethyl acetate/hexane to give 0.85 g of the title compound.

### EXAMPLE 5

### (E)-2-Phenyl-3-(5,6-dichloroindol-3-yl-2-carboxylic acid)-propenoic acid

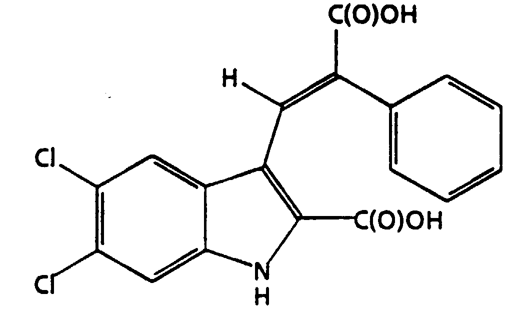

Combine (E) and (Z)-2-phenyl-3-(2-carboethoxy-5,6-dichloroindol-3-yl)-propenoic acid, ethyl ester (850 mg, 1.7 mmol) and lithium hydroxide hydrate (508 mg, 12.1 mmol) in THF/water (20 mL, 1/1) and heat to 50°C for 24 hours. Dilute the reaction mixture with water (50 mL) and acidify with 1M hydrochloric acid. Extract with ethyl acetate and dry the organic layer with magnesium sulfate. Evaporate in vacuo and recrystallize the residue from ethyl acetate/hexane to give 330 mg of the title compound; mp: 272-276°C (dec). Elem. Anal. calculated for C₁₈H₁₁Cl₂NO₄(0.25 ethyl acetate): C, 56.04; H, 3.46; N, 3.45. Found: C, 56.07; H, 3.37; N, 3.60.

### EXAMPLE 6

### (E) and (Z)-N-Methyl-2-phenyl-3-(2-carbomethylamino-4,6-dichloroindol-3-yl)propenoic amide

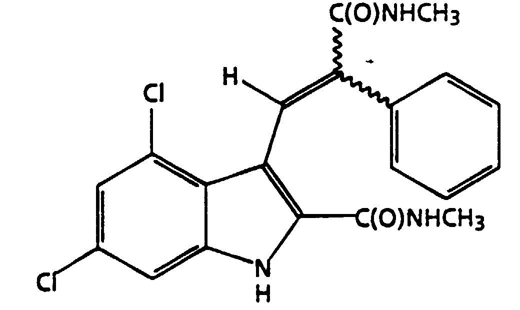

Combine (E) and (Z)-2-phenyl-3-(2-carboethoxy-4,6-dichloroindol-3-yl)propenoic acid, ethyl ester in THF/water (3 mL, 1/1), bubble in an excess of methylamine gas, seal and stir for 24 hours. Dilute the reaction mixture with water (5 mL) and acidify with 1N hydrochloric acid. Extract with ethyl acetate and dry the organic layer with magnesium sulfate. Evaporate in vacuo and recrystallize the residue to give the title compound.

### EXAMPLE 7

### (E) and (Z)-2-Phenyl-3-(2-carboethoxy-6-chloroindol-3-yl)-propenoic acid, ethyl ester

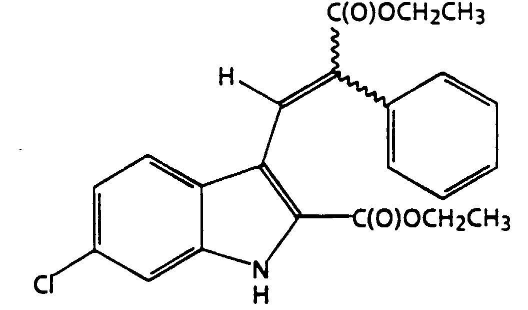

Combine 6-chloro-3-iodoindole-2-carboxylic acid, ethyl ester (0.34g, 0.98 mmol) and 2-phenyl-3-(tri-n-butylstannyl)propenoic acid, ethyl ester (0.44 g, 0.98 mmol) in 1-methyl-2-pyrrolidinone (2 mL) and add bis-(acetonitrile)palladium (II) dichloride (2.07 mg, 0.08 mmol). Flush the vessel with nitrogen gas, seal and heat to 80°C. After stirring for 5 hours pour the reaction mixture into water and extract with ethyl acetate, dry over magnesium sulfate and evaporate in vacuo. Chromatograph on silica gel eluting with 1/6 ethyl acetate/hexane to give 0.13 g of the title compound. R_{f}=0.20, silica gel, 1/6 ethyl acetate/hexane.

### EXAMPLE 8

### (E)-2-Phenyl-3-(6-chloroindol-3-yl-2-carboxylic acid)-propenoic acid

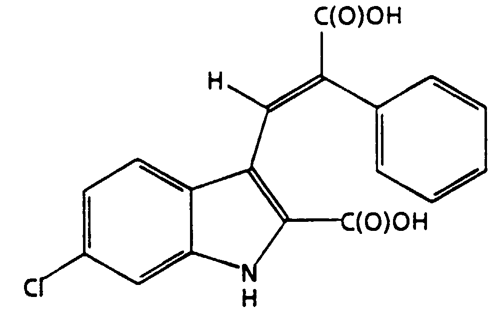

Combine (E) and (Z)-2-phenyl-3-(2-carboethoxy-6-chloroindol-3-yl)propenoic acid, ethyl ester (123 mg, 0.32 mmol) and an aqueous solution of lithium hydroxide hydrate (2.6 mL, 1.0 M, 2.6 mmol) in THF (5 mL) and heat to reflux for 24 hours. Evaporate in vacuo to remove most of the THF. Dilute the reaction mixture with water (50 mL) and extract with ethyl acetate and discard the organic layer. Acidify the aqueous layer with 1M hydrochloric acid. Extract with ethyl acetate (3 X 75 mL), combine the organic layers, and dry over magnesium sulfate. Evaporate in vacuo and recrystallize the residue from ethyl acetate/hexane to give the title compound; mp: 232-236°C (dec).

### EXAMPLE 9

### (E) and (Z)-2-(4-Methoxyphenyl)-3-(2-carboethoxy-4,6-dichloroindol-3-yl)propenoic acid, ethyl ester

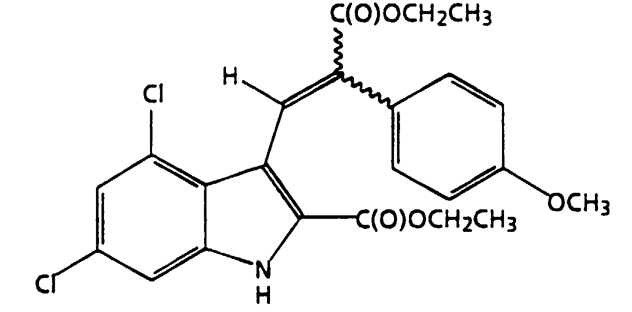

Combine 4,6-dichloro-3-iodoindole-2-carboxylic acid, ethyl ester (4.0 mmol) and 2-(4-methoxyphenyl)-3-(tri-n-butylstannyl)propenoic acid, ethyl ester (2.5 mmol) in 1-methyl-2-pyrrolidinone (5 mL) and add bis-(acetonitrile)palladium (II) dichloride (0.08 mmol). Flush the vessel with nitrogen gas, seal and heat to 60°C. After stirring for 8 hours add more bis(acetonitrile)palladium (II) dichloride (2.07 mg, 0.08 mmol) and continue stirring for 16 hours. Pour the reaction mixture into water and extract with diethyl ether, dry over magnesium sulfate and evaporate in vacuo. Chromatograph on silica gel to give the title compound.

### EXAMPLE 10

### (E) and (Z)-2-(4-Methylphenyl)-3-(2-carboethoxy-4,6-dichloroindol-3-yl)propenoic acid, ethyl ester

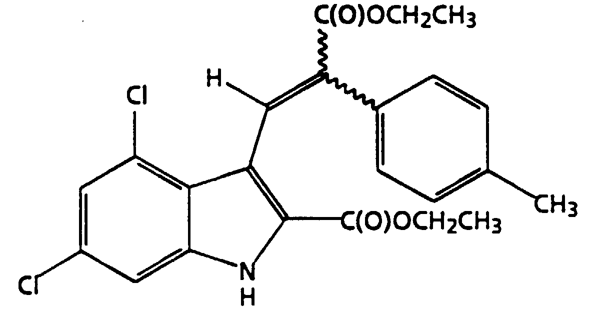

Prepare by the method of Example 9 using 2-(4-methylphenyl)-3-(tri-n-butylstannyl)propenoic acid, ethyl ester.

### EXAMPLE 11

### (E) and (Z)-2-(4-Chlorophenyl)-3-(2-carboethoxy-4,6-dichloroindol-3-yl)propenoic acid, ethyl ester

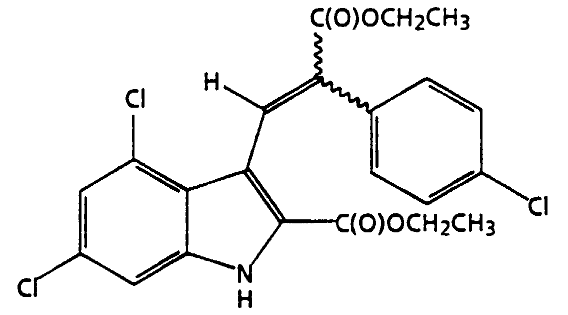

Prepare by the method of Example 9 using 2-(4-chlorophenyl)-3-(tri-n-butylstannyl)propenoic acid, ethyl ester.

### EXAMPLE 12

### (E) and (Z)-2-4-N,N'-(1,1,4,4-Tetramethyl-1,4-disilethyleneaminophenyl)-3-(2-carboethoxy-4,6-dichloro indol-3-yl)propenoic acid, ethyl ester

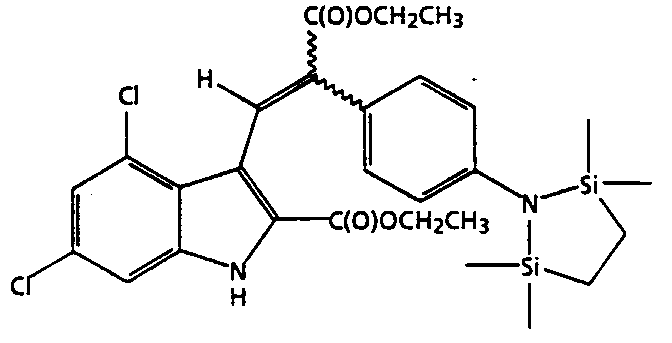

Prepare by the method of Example 9 using 2-4-N,N'-(1,1,4,4-tetramethyl-1,4-disilethyleneaminophenyl)-3-(tri-n-butylstannyl)propenoic acid, ethyl ester.

### EXAMPLE 13

### (E) and (Z)-2-Phenyl-3-(2-carboethoxy-4,6-dichloroindol-3-yl)propenoic acid, t-butyl ester

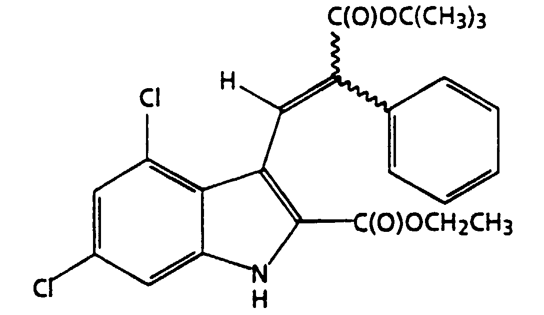

Prepare by the method of Example 9 using 2-phenyl-3-(tri-n-butylstannyl)propenoic acid, t-butyl ester.

### EXAMPLE 14

### (E) and (Z)-2-(4-Methoxyphenyl)-3-(4,6-dichloroindol-3-yl-2-carboxylic acid)propenoic acid

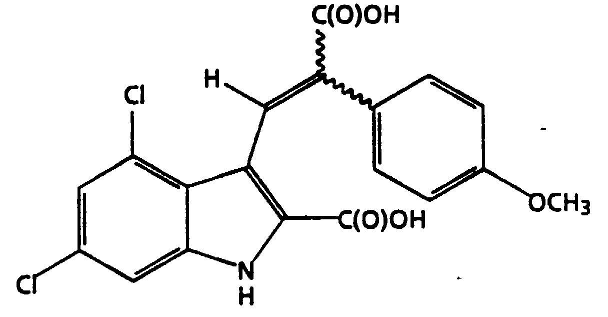

Prepare by the method of Example 3 using (E) and (Z)-2-(4-methoxyphenyl)-3-(2-carboethoxy-4,6-dichloroindol-3-yl)propenoic acid, ethyl ester.

### EXAMPLE 15

### (E) and (Z)-2-(4-Methylphenyl)-3-(4,6-dichloroindol-3-yl-2-carboxylic acid)propenoic acid

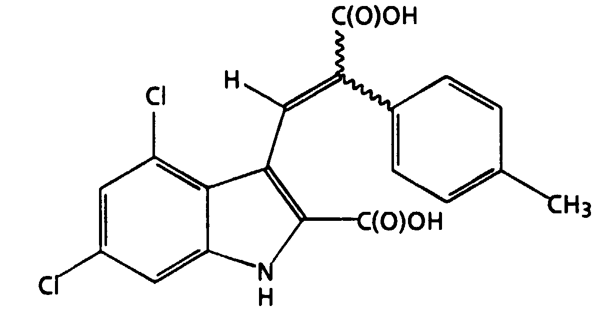

Prepare by the method of Example 3 using (E) and (Z)-2-(4-methylphenyl)-3-(2-carboethoxy-4,6-dichloroindol-3-yl)propenoic acid, ethyl ester.

### EXAMPLE 16

### (E) and (Z)-2-(4-Chlorophenyl)-3-(4,6-dichloroindol-3-yl-2-carboxylic acid)propenoic acid

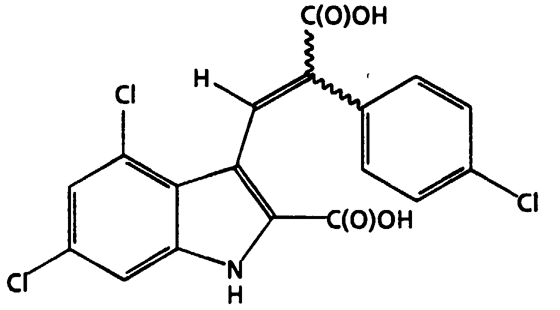

Prepare by the method of Example 3 using (E) and (Z)-2-(4-chlorophenyl)-3-(2-carboethoxy-4,6-dichloroindol-3yl)propenoic acid, ethyl ester.

### EXAMPLE 17

### (E) and (Z)-2-(4-Aminophenyl)-3-(2-carboethoxy-4,6-dichloroindol-3-yl)propenoic acid, ethyl ester

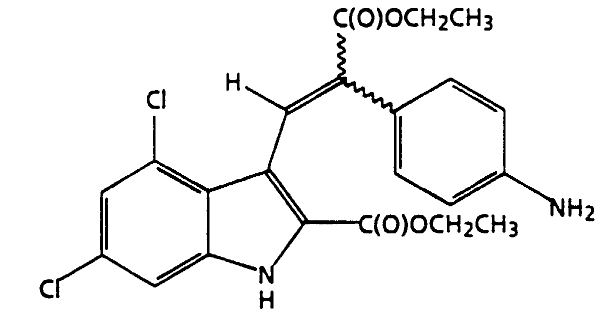

Combine (E) and (Z)-2-4-N,N'-(1,1,4,4-tetramethyl-1,4-disilethyleneaminophenyl)-3-(2-carboethoxy-4,6-dichloro-indol-3-yl)propenoic acid, ethyl ester (2 mmol) and ethanol (10 mL). Cool to 0°C in an ice-bath and add 2 M hydrochloric acid solution (0.5 mL). After 1 hour, partition the reaction mixture between saturated sodium bicarbonate solution and dichloromethane. Extract the aqueous layer with dichloromethane and combine the organic layers. Dry the organic layers over magnesium sulfate, filter, and evaporate in vacuo to give the title compound.

### EXAMPLE 18

### (E) and (Z)-2-(4-Aminophenyl)-3-(4,6-dichloroindol-3-yl-2-carboxylic acid)propenoic acid

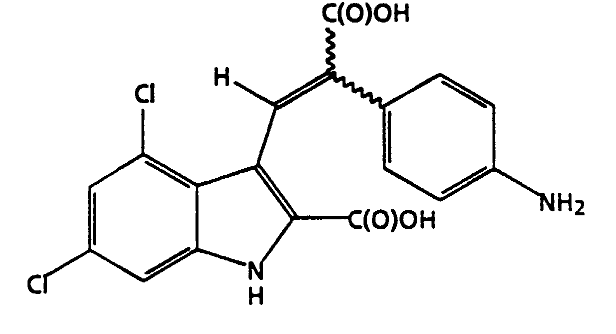

Prepare by the method of Example 3 using (E) and (Z)-2-(4-Aminophenyl)-3-(2-carboethoxy-4,6-dichloroindol-3-yl)propenoic acid, ethyl ester.

### EXAMPLE 19

### (E) and (Z)-2-Phenyl-3-(1-p-toluenesulfonyl-2-carboethoxy-4,6-dichloroindol-3-yl)propenoic acid, t-butyl ester

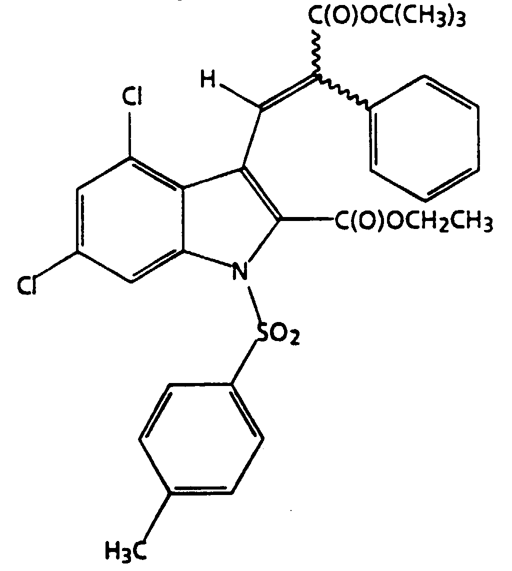

Combine (E) and (Z)-2-phenyl-3-(2-carboethoxy-4,6-dichloroindol-3-yl)propenoic acid, t-butyl ester (5 mmol) and sodium hydride (5 mmol) in tetrahydrofuran (10 mL) and allow to stir until gas evolution ceases. Add p-toluenesulfonyl chloride (6 mmol). After 24 hours, pour the reaction mixture into water and extract with ethyl acetate. Dry the organic layer over magnesium sulfate, filter, and evaporate in vacuo. Chromatograph on silica gel to give the title compound.

### EXAMPLE 20

### (E) and (Z)-2-Phenyl-3-(1-p-toluenesulfonyl-2-carboethoxy-4,6-dichloroindol-3-yl)propenoic acid

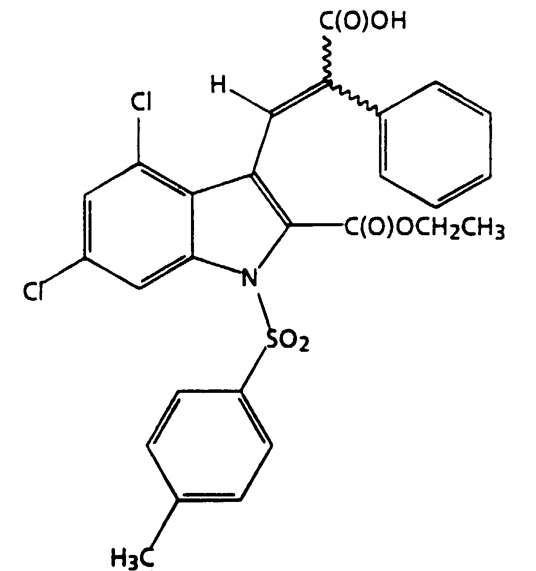

Cool a solution of trifluoroacetic acid (5 mL) and anisole (2 mmol) in dichloromethane (10 mL) in an ice-bath. Add (E) and (Z)-2-phenyl-3-(1-p-toluenesulfonyl-2-carboethoxy-4,6-dichloroindol-3-yl)propenoic acid, t-butyl ester (1 mmol) and stir for 3 hours. Evaporate in vacuo. Repeatedly, add carbon tetrachloride and evaporate in vacuo to remove residual trifluoroacetic acid. Triturate with hexane to give the title compound.

### EXAMPLE 21

### (E) and (Z)-2-Phenyl-3-(1-p-toluenesulfonyl-2-carboethoxy-4,6-dichloroindol-3-yl)propenoic acid chloride

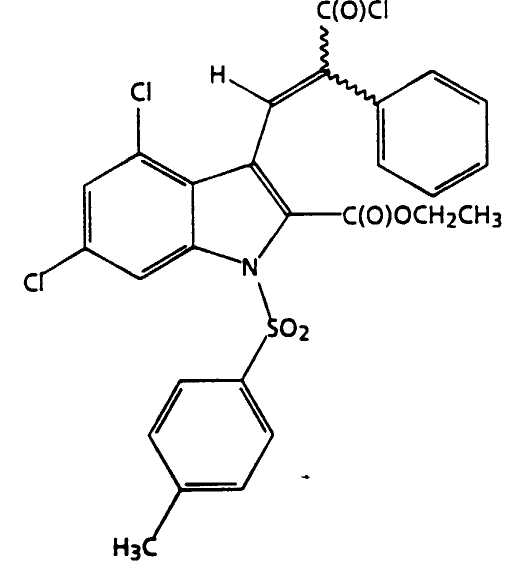

Combine (E) and (Z)-2-phenyl-3-(l-p-toluenesulfonyl-2-carboethoxy-4,6-dichloroindol-3-yl)propenoic acid (5mmol) and oxalyl chloride (20 mL). Add dimethylformamide (0.1 mL) and heat to reflux. After 4 hours, evaporate in vacuo. Add hexane and evaporate in vacuo to give the title compound.

### EXAMPLE 22

### (E) and (Z)-N,N-Dimethyl-2-phenyl-3-(1-p-toluenesulfonyl-4,6-dichloroindol-3-yl-2-carboethoxy)propenoic amide

Combine (E) and (Z)-2-phenyl-3-(1-p-toluenesulfonyl-2-carboethoxy-4,6-dichloroindol-3-yl)propenoic acid chloride and tetrahydrofuran. Cool in an ice-bath. Add triethylamine (6 mmol). Add dimethylamine as a gas by slowly bubbling a stream of dimethylamine gas into the solution for 20 minutes. Warm to ambient temperature and stir for 24 hours. Evaporate in vacuo to give a residue. Partition the residue between 1 M hydrochloric acid solution and ethyl acetate. Extract the aqueous layer with ethyl acetate. Dry the combined organic layers over magnesium sulfate, filter, and evaporate in vacuo. Chromatograph on silica gel to give the title compound.

### EXAMPLE 23

### (E) and (Z)-N-Methyl-2-phenyl-3-(1-p-toluenesulfonyl-4,6-dichloroindol-3-yl-2-carboethoxy)propenoic amide

Prepare by the method of Example 22 using methylamine as a gas.

### EXAMPLE 24

### (E) and (Z)-N-Phenyl-2-phenyl-3-(1-p-toluenesulfonyl-4,6-dichloroindol-3-yl-2-carboethoxy)propenoic amide

Prepare by the method of Example 22 using aniline.

### EXAMPLE 25

### (E) and (Z)-N-Methyl-N-phenyl-Z-phenyl-3-(1-p-toluenesulfonyl-4,6-dichloroindol-3-yl-2 -carboethoxy)propenoic amide

Prepare by the method of Example 22 using N-methylaniline.

### EXAMPLE 26

### (E) and (Z)-N-Benzyl-2-phenyl-3-(1-p-toluenesulfonyl-4,6-dichloroindol-3-yl-2-carboethoxy)propenoic amide

Prepare by the method of Example 22 using benzylamine.

### EXAMPLE 27

### (E) and (Z)-N-Morpholino-2-phenyl-3-(1-p-toluenesulfonyl-4,6-dichloroindol-3-yl-2-carboethoxy)propenoic amide

Prepare by the method of Example 22 using morpholine.

### EXAMPLE 28

### (E) and (Z)-N-4-Methylpiperazino-2-phenyl-3-(1-p-toluenesulfonyl-4,6-dichloroindol-3-yl-2 -carboethoxy)propenoic amide

Prepare by the method of Example 22 using 4-methylpiperazine.

### EXAMPLE 29

### (E) and (Z)-N,N-Dimethyl-2-phenyl-3-(4,6-dichloroindol-3-yl-2-carboxylic acid)propenoic amide

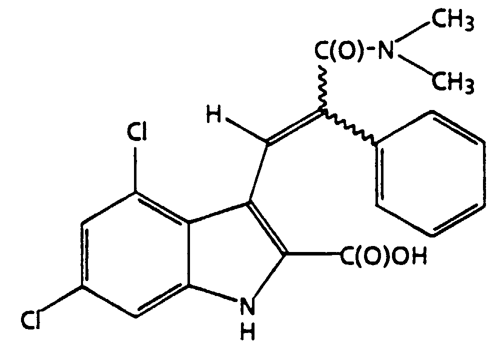

Combine (E) and (Z)-N,N-dimethyl-2-phenyl-3-(1-p-toluenesulfonyl-4,6-dichloroindol-3-yl-2-carboethoxy)propenoic amide (2 mmol) and 2 M potassium hydroxide solution (2 mL). Heat to reflux for 8 hours. Add water (20 mL) and evaporate in vacuo to remove the methanol. Add 2 M hydrochloric acid solution until the pH is 2. Filter, rinse with water. Triturate with diethyl ether, filter, and recrystallize to give the title compound.

### EXAMPLE 30

### (E) and (Z)-N-Methyl-2-phenyl-3-(4,6-dichloroindol-3-yl-2-carboxylic acid)propenoic amide

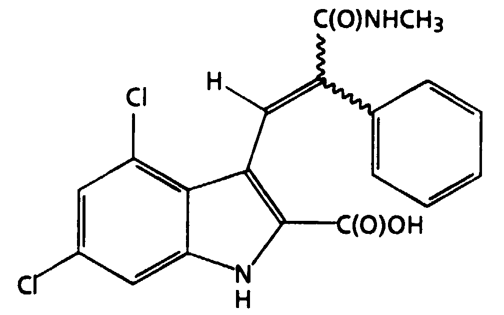

Prepare by the method of Example 29 using (E) and (Z)-N-methyl-2-phenyl-3-(1-p-toluenesulfonyl-4,6-dichloroindol-3-yl-2-carboethoxy)propenoic amide.

### EXAMPLE 31

### (E) and (Z)-N-Phenyl-2-phenyl-3-(4,6-dichloroindol-3-yl-2-carboxylic acid)propenoic amide

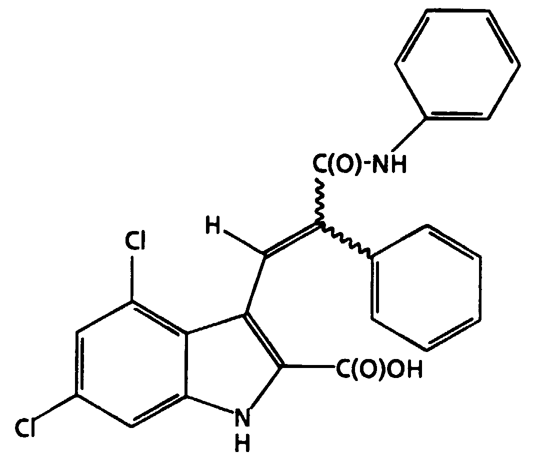

Prepare by the method of Example 29 using (E) and (Z)-N-phenyl-2-phenyl-3-(1-p-toluenesulfonyl-4,6-dichloroindol-3-yl-2-carboethoxy)propenoic amide.

### EXAMPLE 32

### (E) and (Z)-N-Methyl-N-phenyl-2-phenyl-3-(4,6-dichloroindol-3-yl-2-carboxylic acid)propenoic amide

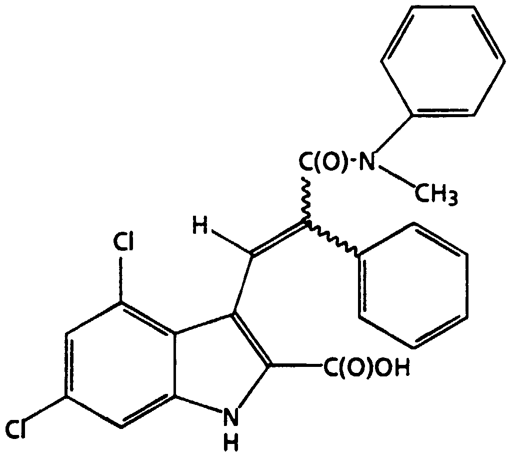

Prepare by the method of Example 29 using (E) and (Z)-N-methyl-N-phenyl-2-phenyl-3-(1-p-toluenesulfonyl-4,6-dichloroindol-3-yl-2-carboethoxy)propenoic amide.

### EXAMPLE 33

### (E) and (Z)-N-Benzyl-2-phenyl-3-(4,6-dichloroindol-3-yl-2-carboxylic acid)propenoic amide

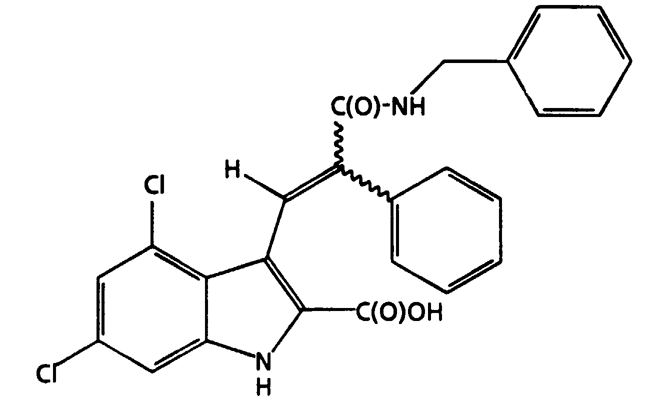

Prepare by the method of Example 29 using (E) and (Z)-N-benzyl-2-phenyl-3-(1-p-toluenesulfonyl-4,6-dichloroindol-3-yl-2-carboethoxy)propenoic amide.

### EXAMPLE 34

### (E) and (Z)-N-Morpholino-2-phenyl-3-(4,6-dichloroindol-3-yl-2-carboxylic acid)propenoic amide

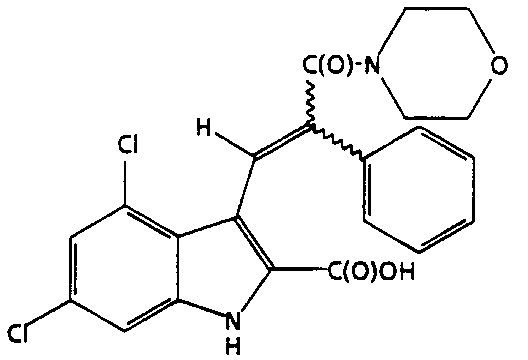

Prepare by the method of Example 29 using (E) and (Z)-N-morpholino-2-phenyl-3-(1-p-toluenesulfonyl-4,6-dichloroindol-3-yl-2-carboethoxy)propenoic amide.

### EXAMPLE 35

### (E) and (Z)-N-4-Methylpiperazino-2-phenyl-3-(4,6-dichloroindol-3-yl-2-carboxylic acid)propenoic amide

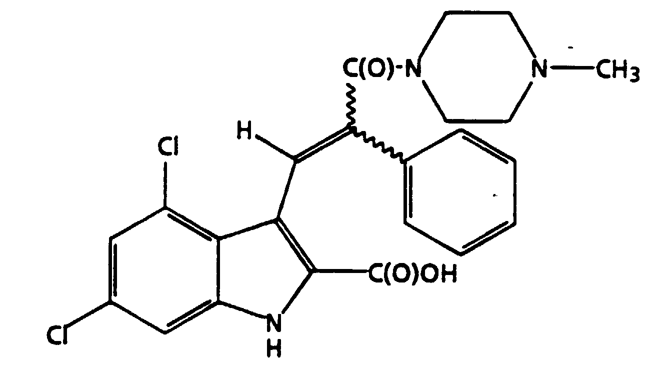

Combine (E) and (Z)-N-4-methylpiperazino-2-phenyl-3-(1-p-toluenesulfonyl-4,6-dichloroindol-3-yl-2-carboethoxy)propenoic amide (2 mmol) and 2 M potassium hydroxide solution (2 mL). Heat to reflux for 8 hours. Add 2 M hydrochloric acid solution until the pH is 2. Evaporate in vacuo to remove the methanol and lyophilize to remove the water to obtain a residue. Triturate the residue repeatedly with ethanol. Evaporate the filtrate in vacuo to obtain a residue. Dissolve the residue in the minimum amount of isopropanol. Add propylene oxide and allow to stand until a solid forms. Filter and rinse with isopropanol to give the title compound.

### EXAMPLE 36

### (E) and (Z)-2-Phenyl-3-(2-carboethoxy-6-chloroindol-3-yl)-propenoic acid, methyl ester

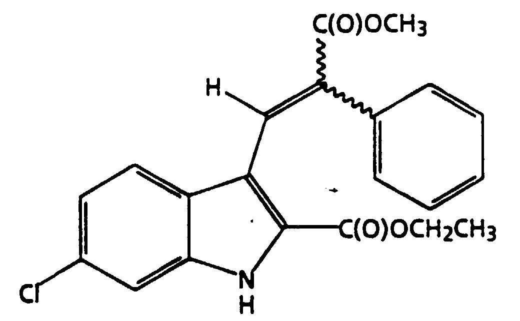

Prepare by the method of Example 7 using 2-phenyl-3-(tri-n-butylstannyl)propenoic acid, methyl ester. Elem. Anal. calculated for C₂₁H₁₈NO₄Cl: C, 65.71; H,4.73; N, 3.65. Found: C, 65.72; H,4.72; N, 3.58.

### EXAMPLE 37

### (E) and (Z)-2-Phenyl-3-(2-carboethoxy-4,6-dichloroindol-3-yl)propenoic acid, methyl ester

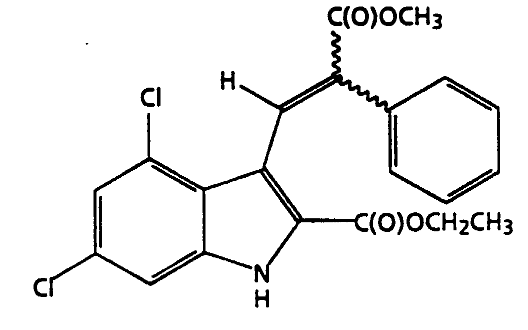

Prepare by the method of Example 1 using 2-phenyl-3-(tri-n-butylstannyl)propenoic acid, methyl ester.

## Claims

1. A compound of the formula: 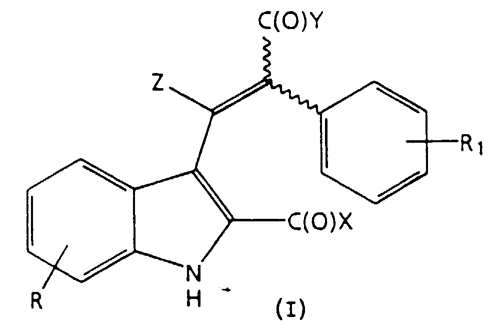 wherein
Z is hydrogen, -CH₃, or -C₂H₅;
X and Y are represented by -OH; -OR_{2,} -OCH₂OR₂ or -O-(CH₂)ₚ-NR₅R₆; R₂ is represented by C₁₋₄ alkyl, phenyl, substituted phenyl, or a phenylalkyl substituent in which the phenyl ring may be optionally substituted; R₅ and R₆ are each independently represented by a C₁₋₄ alkyl or together with the adjacent nitrogen atom form a piperidino, morpholino, or pyrrolidino group; or -NR₃R₄; R₃ and R₄ are each independently represented by hydrogen, phenyl, substituted phenyl, phenylalkyl, or a C₁₋₄ alkyl; or R₃ and R₄ are taken together with the adjacent nitrogen to form a ring -CH₂-CH₂-Z-CH₂-CH₂- wherein Z is a bond, O, S, or -NR₇ in which R₇ is hydrogen or C₁₋₄ alkyl;
R is represented by from 1 to 3 substituents independently chosen from the group: hydrogen, C₁₋₄ alkyl, C₁₋₄ alkoxy, halogen, -CF₃, or -OCF₃;
R₁ is represented by from 1 to 3 substituents independently chosen from the group: hydrogen, amino, C₁₋₄ alkyl, C₁₋₄ alkoxy, halogen, -CF₃, or -OCF₃;
and pharmaceutically acceptable addition salts thereof.

2. A compound of Claim 1 wherein Z is hydrogen.

3. A compound of Claim 2 wherein R is 4,6-dichloro.

4. A compound of Claim 3 wherein X and Y are ethoxy.

5. A compound of Claim 3 wherein X and Y are -OH.

6. A compound of Claim 3 wherein X and Y are -NHCH₃.

7. A compound of Claim 3 wherein X is -OH and Y is a physiologically acceptable amide.

8. A compound of Claim 7 wherein X is -OH and Y is -NHPh.

9. A compound of Claim 2 wherein R is 6-chloro.

10. A compound of Claim 9 wherein X is -OH and Y is a physiologically acceptable amide.

11. A compound of Claim 10 wherein X is -OH and Y is -NHPh.

12. A compound of Claim 9 wherein X and Y are ethoxy.

13. A compound of Claim 9 wherein X and Y are -OH.

14. A compound of Claim 2 wherein R is 5,6-dichloro.

15. A compound of Claim 14 wherein X and Y are ethoxy.

16. A compound of Claim 14 wherein X and Y are -OH.

17. Use of a compound as defined in claim 1 for the preparation of a pharmaceutical composition useful for antagonizing the effects of excitatory amino acids upon the NMDA receptor complex.

18. Use of a compound as defined in claim 1 for the preparation of a pharmaceutical composition useful for the treatment of neurodegenerative diseases.

19. Use of a compound according to claim 1 in the manufacture of a medicament for preventing ischemic/hypoxic/ hypoglycemic damage to cerebral tissue.

20. Use of a compound according to claim 1 in the manufacture of a medicament for the treatment of anxiety.

21. Use of a compound according to claim 1 in the manufacture of a medicament for producing an analgesic effect.

## Patentansprüche

1. Verbindung der Formel 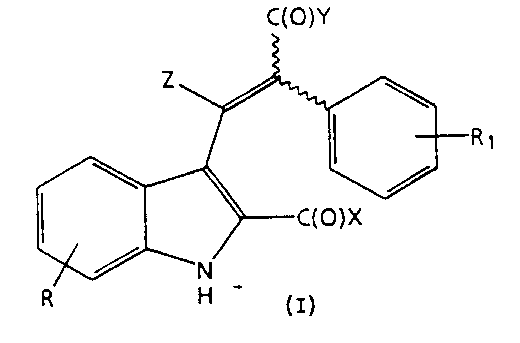 in der
Z ein Wasserstoffatom, eine CH₃-Gruppe oder eine C₂H₅-Gruppe ist;
X und Y für -OH, -OR₂, -OCH₂OR₂ oder -O-(CH₂)ₚ-NR₅R₆ , wobei R₂ für einen C₁₋₄-Alkylrest, Phenylrest, substituierten Phenylrest oder einen Phenylalkylsubstituenten steht, in dem der Phenylring gegebenenfalls substituiert sein kann, R₅ und R₆ jeweils unabhängig voneinander für einen C₁₋₄-Alkylrest stehen oder zusammen mit dem benachbarten Stickstoffatom eine Piperidino-, Morpholino- oder Pyrrolidinogruppe darstellen; oder für -NR₃R₄ stehen, wobei R₃ und R₄ jeweils unabhängig voneinander für ein Wasserstoffatom, einen Phenylrest, substituierten Phenylrest, Phenylalkylrest oder einen C₁₋₄-Alkylrest stehen, oder wobei R₃ und R₄ mit dem benachbarten Stickstoffatom zu einem Ring -CH₂-CH₂-Z-CH₂-CH₂- zusammengefaßt sind, in dem Z eine Bindung, ein Sauerstoffatom, ein Schwefelatom oder ein Rest -NR₇ ist, in dem R₇ für ein Wasserstoffatom oder einen C₁₋₄-Alkylrest steht;
R für 1 bis 3 Substituenten steht, die unabhängig voneinander aus der Gruppe Wasserstoffatom, C₁₋₄-Alkylrest, C₁₋₄Alkoxyrest, Halogenatom, -CF₃ oder -OCF₃ ausgewählt sind;
R₁ für 1 bis 3 Substituenten steht, die unabhängig voneinander aus der Gruppe Wasserstoffatom, Aminogruppe, C₁₋₄-Alkylrest, C₁₋₄-Alkoxyrest, Halogenatom, -CF₃ oder -OCF₃ ausgewählt sind;
sowie pharmazeutisch verträgliche Additionssalze davon.

2. Verbindung nach Anspruch 1, wobei Z ein Wasserstoffatom ist.

3. Verbindung nach Anspruch 2, wobei R für eine 4,6-Dichlor-Substitution steht.

4. Verbindung nach Anspruch 3, wobei X und Y Ethoxygruppen sind.

5. Verbindung nach Anspruch 3, wobei X und Y OH-Gruppen sind.

6. Verbindung nach Anspruch 3, wobei X und Y für -NHCH₃ stehen

7. Verbindung nach Anspruch 3, wobei X eine OH-Gruppe ist und Y ein physiologisch verträglicher Amidrest ist.

8. Verbindung nach Anspruch 7, wobei X eine OH-Gruppe ist und Y für -NHPh steht.

9. Verbindung nach Anspruch 2, wobei R ein 6-Chlor-Substituent ist.

10. Verbindung nach Anspruch 9, wobei X eine OH-Gruppe ist und Y ein physiologisch verträglicher Amidrest ist.

11. Verbindung nach Anspruch 10, wobei X eine OH-Gruppe ist und Y für -NHPh steht.

12. Verbindung nach Anspruch 9, wobei X und Y Ethoxygruppen sind.

13. Verbindung nach Anspruch 9, wobei X und Y OH-Gruppen sind.

14. Verbindung nach Anspruch 2, wobei R für eine 5,6-Dichlor-Substitution steht.

15. Verbindung nach Anspruch 14, wobei X und Y Ethoxygruppen sind.

16. Verbindung nach Anspruch 14, wobei X und Y OH-Gruppen sind.

17. Verwendung einer wie in Anspruch 1 definierten Verbindung für die Herstellung eines Arzneimittels, das als Antagonist gegen die Wirkungen exzitatorischer Aminosäuren auf den NMDA-Rezeptorkomplex nützlich ist.

18. Verwendung einer wie in Anspruch 1 definierten Verbindung für die Herstellung eines Arzneimittels, das bei der Behandlung neurodegenerativer Erkrankungen nützlich ist.

19. Verwendung einer Verbindung nach Anspruch 1 bei der Herstellung eines Medikamentes zur Verhütung ischämischer / hypoxischer / hypoglycämischer Schäden an zerebralem Gewebe.

20. Verwendung einer Verbindung nach Anspruch 1 bei der Herstellung eines Medikamentes zur Behandlung von Angstzuständen.

21. Verwendung einer Verbindung nach Anspruch 1 bei der Herstellung eines Medikamentes zur Erzeugung einer analgetischen Wirkung.

## Revendications

1. Composé de formule 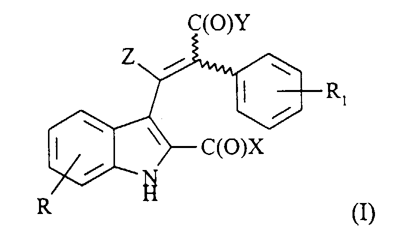 dans laquelle
Z est un atome d'hydrogène ou un groupe -CH₃ ou -C₂H₅;
X et Y représentent -OH, -OR₂, -OCH₂OR₂ ou -O-(CH₂)ₚ-NR₅R₆; R₂ représente un substituant alkyle en C₁-C₄, phényle, phényle substitué ou phénylalkyle dans lequel le noyau phényle peut éventuellement être substitué; R₅ et R₆ représentent chacun indépendamment un groupe alkyle en C₁-C₄, ou forment ensemble, avec l'atome d'azote adjacent, un groupe pipéridino, morpholino ou pyrrolidino; ou -NR₃R₄; R₃ et R₄ représentent chacun indépendamment un atome d'hydrogène ou un groupe phényle, phényle substitué, phénylalkyle ou alkyle en C₁-C₄; ou R₃ et R₄ sont pris ensemble avec l'atome d'azote adjacent pour former un cycle -CH₂-CH₂-Z-CH₂-CH₂- dans lequel Z est une liaison, O, S ou -NR₇, où R₇ est un atome d'hydrogène ou un groupe alkyle en C₁-C₄;
R représente 1 à 3 substituants choisis indépendamment dans le groupe constitué par l'hydrogène et les groupes alkyle en C₁-C₄, alcoxy en C₁-C₄, halogène, -CF₃ ou -OCF₃;
R₁ représente 1 à 3 substituants choisis indépendamment dans le groupe constitué par l'hydrogène et les groupes amino, alkyle en C₁-C₄, alcoxy en C₁-C₄, halogène, -CF₃ ou -OCF₃;
et leurs sels d'addition pharmaceutiquement acceptables.

2. Composé selon la revendication 1, dans lequel Z est l'hydrogène.

3. Composé selon la revendication 2, dans lequel R est 4,6-dichloro.

4. Composé selon la revendication 3, dans lequel X et Y sont des groupes éthoxy.

5. Composé selon la revendication 3, dans lequel X et Y sont des groupes -OH.

6. Composé selon la revendication 3, dans lequel X et Y sont des groupes -NHCH₃.

7. Composé selon la revendication 3, dans lequel X est -OH et Y est un amide physiologiquement acceptable.

8. Composé selon la revendication 7, dans lequel X est -OH et Y est -NHPh.

9. Composé selon la revendication 2, dans lequel R est 6-chloro.

10. Composé selon la revendication 9, dans lequel X est -OH et Y est un amide physiologiquement acceptable.

11. Composé selon la revendication 10, dans lequel X est -OH et Y est -NHPh.

12. Composé selon la revendication 9, dans lequel X et Y sont des groupes éthoxy.

13. Composé selon la revendication 9, dans lequel X et Y sont des groupes -OH.

14. Composé selon la revendication 2, dans lequel R est 5,6-dichloro.

15. Composé selon la revendication 14, dans lequel X et Y sont des groupes éthoxy.

16. Composé selon la revendication 14, dans lequel X et Y sont des groupes -OH.

17. Utilisation d'un composé tel que défini dans la revendication 1 pour la préparation d'une composition pharmaceutique utile comme antagoniste des effets d'aminoacides excitateurs sur le complexe du récepteur de NMDA.

18. Utilisation d'un composé tel que défini dans la revendication 1 pour la préparation d'une composition pharmaceutique utile pour le traitement de maladies neurodégénératives.

19. Utilisation d'un composé selon la revendication 1 dans la fabrication d'un médicament utile pour la prévention de lésions ischémiques/hypoxiques/ hypoglycémiques du tissu cérébral.

20. Utilisation d'un composé selon la revendication 1 dans la fabrication d'un médicament utile pour le traitement de l'anxiété.

21. Utilisation d'un composé selon la revendication 1 dans la fabrication d'un médicament produisant un effet analgésique.
